(19) Europäisches Patentamt
European Patent Office
Office européen des brevets

(11) Veröffentlichungsnummer: **0 287 851**
**A1**

(12) **EUROPÄISCHE PATENTANMELDUNG**

(21) Anmeldenummer: **88104950.6**

(22) Anmeldetag: **28.03.88**

(51) Int. Cl.⁴: **C07D 231/14 , C07D 401/04 , A01N 43/56**

(30) Priorität: **09.04.87 DE 3711928**

(43) Veröffentlichungstag der Anmeldung:
**26.10.88 Patentblatt 88/43**

(84) Benannte Vertragsstaaten:
**AT BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Jensen-Korte, Uta, Dr.**
**Geibelstrasse 9**
**D-4000 Düsseldorf 1(DE)**
Erfinder: **Gehring, Reinhold, Dr.**
**Dasnoeckel 49**
**D-5600 Wuppertal 11(DE)**
Erfinder: **Schallner, Otto, Dr.**
**Noldeweg 22**
**D-4019 Monheim(DE)**
Erfinder: **Stetter, Jörg, Dr.**
**Gellertweg 4**
**D-5600 Wuppertal 1(DE)**
Erfinder: **Becker, Benedikt, Dr.**
**Metzkausener Strasse 14**
**D-4020 Mettmann(DE)**
Erfinder: **Homeyer, Bernhard, Dr.**
**Obere Strasse 28**
**D-5090 Leverkusen 3(DE)**

(54) **Substituierte 1-Arylpyrazole.**

(57) Es werden neue 1-Arylpyrazole der Formel

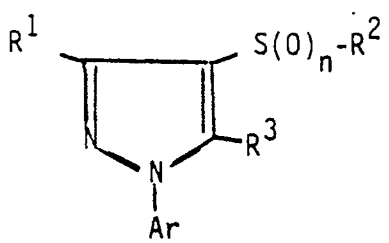

(I)

in welcher
$R^1$ für Wasserstoff, Alkyl oder Halogenalkyl steht,
$R^2$ für Alkyl, Alkenyl, Alkinyl, Cycloalkyl, Halogenalkyl, Halogenalkenyl, Alkoxyalkyl, Alkylthioalkyl, Alkylsulfinylalkyl, Alkylsulfonylalkyl, für gegebenenfalls substituiertes Aralkyl oder für gegebenenfalls substituiertes Aryl steht,
n für die Zahlen 0, 1 oder 2 steht,

EP 0 287 851 A1

$R^3$ für Cyano, für einen Rest

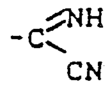

oder für einen Rest $-\underset{\overset{\|}{O}}{C}-Y-R^4$ steht und

Ar für substituiertes Phenyl oder für gegebenenfalls substituiertes Pyridyl steht, wobei

Y für Sauerstoff, Schwefel oder einen Rest $-\underset{|}{N}-R^5$ steht

und wobei

Y, $R^4$ und $R^5$ die im Anmeldungstext angegebene Bedeutung besitzen.

Die in Rede stehenden neuen Verbindungen werden nach an sich bekannten Verfahren hergestellt und besitzen eine ausgezeichnete prestizide und insbesondere insektizide Wirksamkeit.

## Substituierte 1-Arylpyrazole

Die Erfindung betrifft neue substituierte 1-Arylpyrazole, mehrere Verfahren zu ihrer Herstellung sowie ihre Verwendung als Schädlingsbekämpfungsmittel.

Es ist bereits bekannt, daß bestimmte 1-Arylpyrazole, wie beispielsweise das 5-Amino-1-(2,6-dichlor-4-trifluormethylphenyl)-5-methylthio-pyrazol oder das 3-Methyl-4-fluordichlormethyl-sulfonyl-1-(2,6-dichlor-4-trifluormethylphenyl)-5-propionamido-pyrazol gute insektizide Wirksamkeit besitzen (vergl. EP-A 201 852).

Die Wirkung dieser vorbekannten Verbindungen ist jedoch nicht in allen Anwendungsbereichen völlig zufriedenstellend.

Es wurden neue substituierte 1-Arylpyrazole der allgemeinen Formel (I),

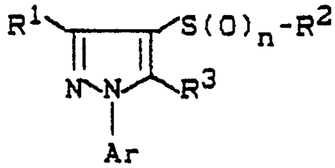

(I)

in welcher

$R^1$ für Wasserstoff, Alkyl oder Halogenalkyl steht,

$R^2$ für Alkyl, Alkenyl, Alkinyl, Cycloalkyl, Halogenalkyl, Halogenalkenyl, Alkoxyalkyl, Alkylthioalkyl, Alkylsulfinylalkyl, Alkylsulfonylalkyl, für gegebenenfalls substituiertes Aralkyl oder für gegebenenfalls substituiertes Aryl steht,

n für die Zahlen 0, 1 oder 2 steht,

$R^3$ für Cyano, für einen Rest

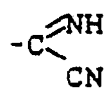

oder für einen Rest $-\underset{\underset{O}{\|}}{C}-Y-R^4$ steht und

Ar für substituiertes Phenyl oder für gegebenenfalls substituiertes Pyridyl steht, wobei

Y für Sauerstoff, Schwefel oder einen Rest $-\underset{|}{N}-R^5$ steht

$R^4$ für Wasserstoff, für Alkyl, Hydroxyalkyl, Alkoxyalkyl, Alkylthioalkyl, Halogenalkyl, Alkenyl, Halogenalkenyl, Alkinyl oder für jeweils gegebenenfalls substituiertes Cycloalkyl, Aralkyl oder Aryl steht,

für einen Rest $-A-\underset{\underset{O}{\|}}{C}-Z-R^6$ steht, oder für den Fall, daß Y für Sauerstoff, Schwefel oder einen Rest

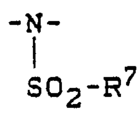

steht, auch für ein salzartig gebundenes Kation steht,

$R^5$ für Wasserstoff, für Alkyl, Hydroxyalkyl, Alkoxyalkyl, Alkylthioalkyl, Halogenalkyl, Alkenyl, Alkinyl oder Halogenalkenyl, für jeweils gegebenenfalls substituiertes Cycloalkyl, Aralkyl oder Aryl steht oder für den Rest $-SO_2R^7$ steht,

A für einen zweifach verknüpften Alkenylrest steht,

Z für Sauerstoff, Schwefel oder einen N-Alkylrest steht,

$R^6$ für Wasserstoff, Alkyl, Alkenyl oder Alkinyl steht und

$R^7$ für jeweils gegebenenfalls substituiertes Alkyl, Aralkyl oder Aryl steht,

gefunden.

Weiterhin wurde gefunden, daß man die neuen substituierten 1-Arylpyrazole der allgemeinen Formel (I),

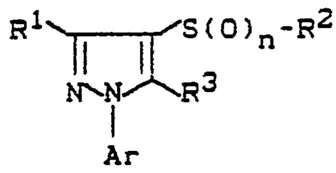

(I)

in welcher
$R^1$ für Wasserstoff, Alkyl oder Halogenalkyl steht,
$R^2$ für Alkyl, Alkenyl, Alkinyl, Cycloalkyl, Halogenalkyl, Halogenalkenyl, Alkoxyalkyl, Alkylthioalkyl, Alkylsulfinylalkyl, Alkylsulfonylalkyl, für gegebenenfalls substituiertes Aralkyl oder für gegebenenfalls substituiertes Aryl steht,
n für die Zahlen 0, 1 oder 2 steht,
$R^3$ für Cyano, für einen Rest

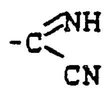

oder für einen Rest $-\underset{\overset{\|}{O}}{C}-Y-R^4$ steht und
Ar für substituiertes Phenyl oder für gegebenenfalls substituiertes Pyridyl steht, wobei
Y für Sauerstoff, Schwefel oder einen Rest $-\underset{|}{N}-R^5$ steht
$R^4$ für Wasserstoff, für Alkyl, Hydroxyalkyl, Alkoxyalkyl, Alkylthioalkyl, Halogenalkyl, Alkenyl, Halogenalkenyl, Alkinyl oder für jeweils gegebenenfalls substituiertes Cycloalkyl, Aralkyl oder Aryl steht,
für einen Rest $-A-\underset{\overset{\|}{O}}{C}-Z-R^6$ steht, oder für den Fall, daß Y für Sauerstoff, Schwefel oder einen Rest

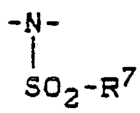

steht, auch für ein salzartig gebundenes Kation steht,
$R^5$ für Wasserstoff, für Alkyl, Hydroxyalkyl, Alkoxyalkyl, Alkylthioalkyl, Halogenalkyl, Alkenyl, Alkinyl oder Halogenalkenyl, für jeweils gegebenenfalls substituiertes Cycloalkyl, Aralkyl oder Aryl steht oder für den Rest $-SO_2-R^7$ steht,
A für einen zweifach verknüpften Alkenylrest steht,
Z für Sauerstoff, Schwefel oder einen N-Alkylrest steht,
$R^6$ für Wasserstoff, Alkyl, Alkenyl oder Alkinyl steht und
$R^7$ für jeweils gegebenenfalls substituiertes Alkyl, Aralkyl oder Aryl steht,
nach einem der im Folgenden beschriebenen Verfahren erhält:
(a) Man erhält substituierte 1-Arylpyrazole der Formel (Ia),

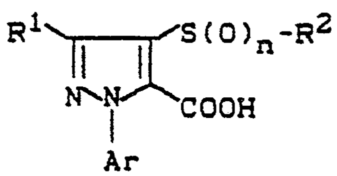

(Ia)

in welcher
$R^1$,$R^2$,Ar und n die oben angegebene Bedeutung haben, wenn man 5-Halogen-1-arylpyrazole der Formel (II),

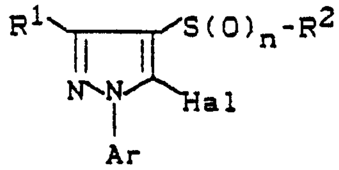

(II)

in welcher
$R^1$,$R^2$,Ar und n die oben angegebene Bedeutung haben und
Hal für Halogen steht,
mit Kohlendioxid in Gegenwart einer Lithium-organischen Verbindung und in Gegenwart eines Verdünnungsmittels umsetzt;

(b) man erhält substituierte 1-Arylpyrazole der Formel (Ib),

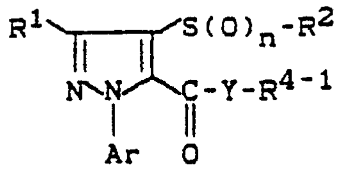

(Ib)

in welcher
$R^1$,$R^2$,Ar,Y und n die oben angegebene Bedeutung haben und
$R^{4-1}$ für Alkyl, Hydroxyalkyl, Alkoxyalkyl, Alkylthioalkyl, Halogenalkyl, Alkenyl, Halogenalkenyl, Alkinyl, für jeweils gegebenenfalls substituiertes Cycloalkyl, Aralkyl oder Aryl steht oder für einen Rest $-A-\underset{\overset{\|}{O}}{C}-Z-R^6$ steht,

wobei
$R^6$, A und Z die oben angegebene Bedeutung haben, und außerdem für den Fall, daß Y für Schwefel oder einen Rest $-\underset{|}{N}-R^5$ steht,
auch für Wasserstoff steht;
wenn man die nach Verfahren (a) erhältlichen substituierten 1-Arylpyrazole der Formel (Ia),

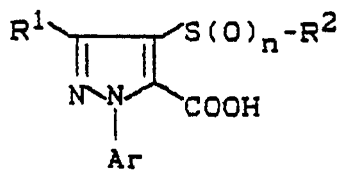

(Ia)

in welcher
$R^1$,$R^2$,Ar und n die oben angegebene Bedeutung haben,
mit Alkoholen, Aminen oder Thiolen der Formel (III),

$R^{4-1}$-Y-H (III)

in welcher
$R^{4-1}$ und Y die oben angegebene Bedeutung haben,
gegebenenfalls in Gegenwart eines Reaktionshilfsmittels und gegebenenfalls in Gegenwart eines Verdünnungsmittels sowie gegebenenfalls in Gegenwart eines Säurebindemittels umsetzt;

(c) man erhält substituierte 1-Arylpyrazole der Formel (Ic),

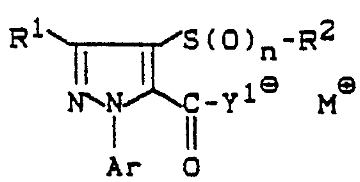

(Ic)

in welcher
$R^1$,$R^2$,Ar und n die oben angegebene Bedeutung haben,
$Y^1$ für Sauerstoff, Schwefel oder einen Rest

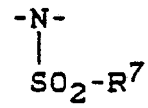

steht und
$M^{\oplus}$ für ein anorganisches oder organisches Kation steht,
wenn man die nach Verfahren (a) oder (b) erhältlichen substituierten 1-Arylpyrazole der Formel (Ia1),

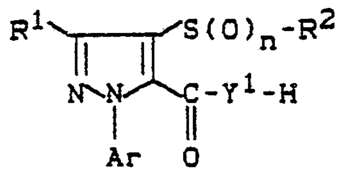

(Ia1)

in welcher
$R^1$,$R^2$,Ar,$Y^1$ und n die oben angegebene Bedeutung haben,
mit anorganischen oder organischen Basen gegebenenfalls in Gegenwart eines Verdünnungsmittels umsetzt;

(d) man erhält substituierte 1-Arylpyrazole der Formel (Id),

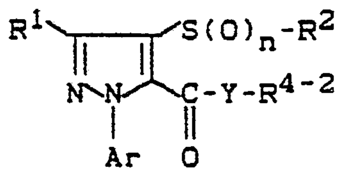

(Id)

in welcher
$R^1$,$R^2$,Ar,Y und n die oben angegebene Bedeutung haben und
$R^{4-2}$ für Alkyl, Hydroxyalkyl, Alkoxyalkyl, Alkylthioalkyl, Halogenalkyl, Alkenyl, Halogenalkenyl, Alkinyl, für jeweils gegebenenfalls substituiertes Cycloalkyl, Aralkyl oder Aryl steht oder für einen Rest $-A-\underset{\underset{O}{\|}}{C}-Z-R^6$ steht, wobei $R^6$, A und Z die oben angegebene Bedeutung haben,
wenn man die nach Verfahren (c) erhältlichen substituierten 1-Arylpyrazole der Formel (Ic),

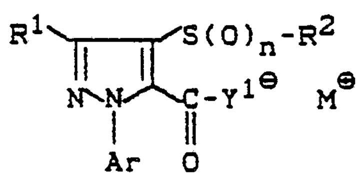

(Ic)

in welcher
$R^1$,$R^2$,Ar,$Y^1$,$M^{\oplus}$ und n die oben angegebene Bedeutung haben,
mit Alkylierungsmitteln der Formel (IV),

$R^{4-2}$-E (IV)

in welcher
$R^{4-2}$ die oben angegebene Bedeutung hat und
E für eine elektronenanziehende Abgangsgruppe steht,
gegebenenfalls in Gegenwart eines Verdünnungsmittels umsetzt;

(e) man erhält substituierte 1-Arylpyrazole der Formel (Ie),

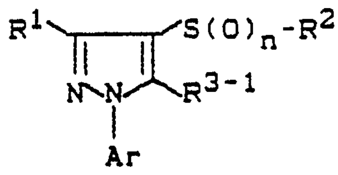

(Ie)

in welcher
$R^1$,$R^2$,Ar und n die oben angegebene Bedeutung haben und
$R^{3-1}$ für Cyano oder für einen Rest

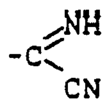

steht,
wenn man 5-Halogen-1-arylpyrazole der Formel (II),

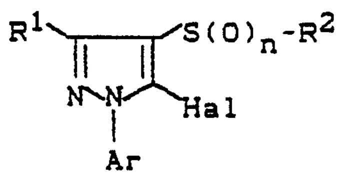

(II)

in welcher
$R^1$,$R^2$,Ar und n die oben angegebene Bedeutung haben,
mit Dicyan in Gegenwart einer Lithium-organischen Verbindung und in Gegenwart eines Verdünnungsmittels umsetzt;

(f) man erhält substituierte 1-Arylpyrazole der Formel (If),

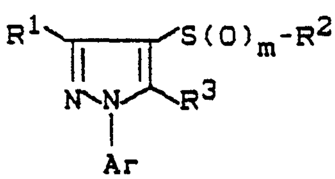

(If)

in welcher
$R^1$,$R^2$,$R^3$ und Ar die oben angegebene Bedeutung haben und
m für eine Zahl 1 oder 2 steht,
wenn man die nach Verfahren (a), (b), (c), (d) oder (e) erhältlichen substituierten 1-Arylpyrazole der Formel (Ig),

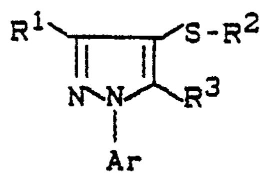

(Ig)

in welcher
$R^1$,$R^2$,$R^3$ und Ar die oben angegebene Bedeutung haben,
mit Oxidationsmitteln gegebenenfalls in Gegenwart eines Verdünnungsmittels und gegebenenfalls in Gegenwart eines Katalysators oxidiert.

Schließlich wurde gefunden, daß die neuen substituierten 1-Arylpyrazole der allgemeinen Formel (I) pestizide und insbesondere insektizide Eigenschaften besitzen.

Überraschenderweise zeigen die erfindungsgemäßen substituierten 1-Arylpyrazole der allgemeinen

Formel (I) eine erheblich bessere insektizide Wirksamkeit als die aus dem Stand der Technik bekannten 1-Arylpyrazole, wie beispiels weise das 5-Amino-1-(2,6-dichlor-4-trifluormethylphenyl)-5-methylthiopyrazol oder das 3-Methyl-4-fluordichlormethylsulfonyl-1-(2,6-dichlor-4-trifluormethylphenyl)-5-propionamido-pyrazol, welches chemisch und wirkungsmäßig naheliegende Verbindungen sind.

Die erfindungsgemäßen substituierten 1-Arylpyrazole sind durch die Formel (I) allgemein definiert.

Bevorzugt sind Verbindungen der Formel (I), bei welchen

$R^1$ für Wasserstoff, oder für jeweils geradkettiges oder verzweigtes Alkyl oder Halogenalkyl mit 1 bis 4 Kohlenstoffatomen und gegebenenfalls 1 bis 9 gleichen oder verschiedenen Halogenatomen steht,

$R^2$ für jeweils geradkettiges oder verzweigtes Alkyl, Alkenyl oder Alkinyl mit jeweils bis zu 8 Kohlenstoffatomen, für Cycloalkyl mit 3 bis 7 Kohlenstoffatomen, für jeweils geradkettiges oder verzweigtes Halogenalkyl oder Halogenalkenyl mit jeweils bis zu 8 Kohlenstoffatomen und bis zu 17 gleichen oder verschiedenen Halogenatomen, für jeweils geradkettiges oder verzweigtes Alkoxyalkyl, Alkylthioalkyl, Alkylsulfinylalkyl oder Alkylsulfonylalkyl mit jeweils 1 bis 6 Kohlenstoffatomen in den einzelnen Alkylteilen oder für jeweils im Phenylteil gegebenenfalls einfach oder mehrfach, gleich oder verschieden substituiertes Phenylalkyl oder Phenyl mit gegebenenfalls 1 bis 4 Kohlenstoffatomen im geradkettigen oder verzweigten Alkylteil steht, wobei als Substituenten im Phenylteil infrage kommen: Halogen, Cyano, Nitro, jeweils geradkettiges oder verzweigtes Alkyl, Alkoxy, Alkylthio, Alkylsulfinyl, Alkylsulfonyl, Halogenalkyl, Halogenalkoxy, Halogenalkylthio, Halogenalkylsulfinyl oder Halogenalkylsulfonyl mit jeweils 1 bis 4 Kohlenstoffatomen in den einzelnen Alkylteilen und gegebenenfalls 1 bis 9 gleichen oder verschiedenen Halogenatomen,

n für eine Zahl 0, 1 oder 2 steht,

$R^3$ für Cyano, für einen Rest

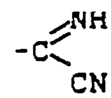

oder für einen Rest $-\underset{\overset{\|}{O}}{C}-X-R^4$ steht und

Ar für einfach oder mehrfach, gleich oder verschieden substituiertes Phenyl oder für jeweils gegebenenfalls einfach oder mehrfach gleich oder verschieden substituiertes 2-Pyridyl, 3-Pyridyl oder 4-Pyridyl steht, wobei als Substituenten jeweils in Frage kommen: Cyano, Nitro, Halogen, jeweils geradkettiges oder verzweigtes Alkyl, Alkoxy oder Alkoxycarbonyl mit jeweils 1 bis 4 Kohlenstoffatomen, außerdem jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $-S(O)_p-R^8$,

wobei

Y für Sauerstoff, Schwefel oder für einen Rest $-\underset{|}{N}-R^5$ steht,

$R^4$ für Wasserstoff, für jeweils geradkettiges oder verzweigtes Alkyl, Hydroxyalkyl, Alkoxyalkyl, Alkylthioalkyl, Halogenalkyl, Alkenyl, Halogenalkenyl oder Alkinyl mit jeweils bis zu 12 Kohlenstoffatomen und im Fall des Halogenalkyl bzw. des Halogenalkenyl mit 1 bis 15 gleichen oder verschiedenen Halogenatomen, für Cycloalkyl mit 3 bis 7 Kohlenstoffatomen oder für jeweils gegebenenfalls einfach oder mehrfach, gleich oder verschieden durch Halogen, Cyano, Nitro sowie jeweils geradkettiges oder verzweigtes Alkyl, Alkoxy, Alkylthio oder Halogenalkyl mit jeweils 1 bis 4 Kohlenstoffatomen und im Fall des Halogenalkyls mit 1 bis 9 gleichen oder verschiedenen Halogenatomen substituiertes Phenyl, Benzyl oder Phenethyl steht, außerdem für einen Rest $-A-\underset{\overset{\|}{O}}{C}-Z-R^6$ steht oder

für den Fall, daß Y für Sauerstoff, Schwefel oder einen Rest $\underset{SO_2-R^7}{\overset{-N-}{|}}$ steht,

auch für ein Äquivalent eines Alkali-, Erdalkali-, Kupfer-, Zink-, Mangan-, Zinn-, Eisen-, Cobalt-oder Nickelkations oder für ein gegebenenfalls einfach oder mehrfach, gleich oder verschieden substituiertes Ammonium-, Phosphonium-oder Sulfoniumkation steht, wobei als Substituenten in Frage kommen: geradkettiges oder verzweigtes Alkyl mit 1 bis 18 Kohlenstoffatomen, Phenyl oder Benzyl,

$R^5$ für Wasserstoff, für jeweils geradkettiges oder verzweigtes Alkyl, Hydroxyalkyl, Alkoxyalkyl, Alkylthioalkyl, Halogenalkyl, Alkenyl, Halogenalkenyl oder Alkinyl mit jeweils bis zu 12 Kohlenstoffatomen und im Falle des Halogenalkyl bzw. des Halogenalkenyl mit 1 bis 15 gleichen oder verschiedenen Halogenatomen, für Cycloalkyl mit 3 bis 7 Kohlenstoffatomen oder für jeweils gegebenenfalls einfach oder mehrfach, gleich oder verschieden durch Halogen, Cyano, Nitro sowie jeweils geradkettiges oder verzweigtes Alkyl, Alkoxy,

Alkylthio oder Halogenalkyl mit jeweils 1 bis 4 Kohlenstoffatomen und im Fall des Halogenalkyls mit 1 bis 9 gleichen oder verschiedenen Halogenatomen substituiertes Phenyl, Benzyl oder Phenethyl steht, außerdem für einen Rest $-SO_2-R^7$ steht,

A für einen zweifach verknüpften geradkettigen oder verzweigten Alkylrest mit 1 bis 8 Kohlenstoffatomen steht,

Z für Sauerstoff, Schwefel oder einen N-Alkylrest mit 1 bis 4 Kohlenstoffatomen im geradkettigen oder verzweigten Alkylteil steht,

$R^6$ für Wasserstoff oder für jeweils geradkettiges oder verzweigtes Alkyl, Alkenyl oder Alkinyl mit jeweils bis zu 6 Kohlenstoffatomen steht,

$R^7$ für jeweils geradkettiges oder verzweigtes Alkyl oder Halogenalkyl mit jeweils 1 bis 6 Kohlenstoffatomen und im Fall des Halogenalkyls mit 1 bis 9 gleichen oder verschiedenen Halogenatomen oder für gegebenenfalls einfach oder mehrfach, gleich oder verschieden substituiertes Phenyl, Benzyl oder Phenylethyl steht, wobei als Substituenten jeweils in Frage kommen: Halogen, Cyano, Nitro, jeweils geradkettiges oder verzweigtes Alkyl, Alkoxy, Alkylthio oder Halogenalkyl mit jeweils 1 bis 4 Kohlenstoffatomen und im fall des Halogenalkyl mit 1 bis 9 gleichen oder verschiedenen Halogenatomen,

$R^8$ für Amino sowie für jeweils geradkettiges oder verzweigtes Alkyl, Alkylamino, Dialkylamino oder Halogenalkyl mit jeweils 1 bis 4 Kohlenstoffatomen in den einzelnen Alkylteilen und im Fall des Halogenalkyl mit 1 bis 9 gleichen oder verschiedenen Halogenatomen steht und

p für eine Zahl 0, 1 oder 2 steht.

Besonders bevorzugt sind substituierte 1-Arylpyrazole der allgemeinen Formel (I), bei welchen

$R^1$ für Wasserstoff, Methyl, Ethyl, n-oder i-Propyl, n-, i-, s-oder t-Butyl oder Trifluormethyl steht,

$R^2$ für Methyl, Ethyl, n-oder i-Propyl, n-, i-, s-oder t-Butyl, n-oder i-Pentyl, n-oder i-Hexyl, Allyl, n-oder i-Butenyl, n-oder i-Pentenyl, Propargyl, n-oder i-Butinyl, Cyclopropyl, Cyclobutyl, Cyclopentyl, Cyclohexyl, Chlormethyl, Difluormethyl, Difluorchlormethyl, Fluordichlormethyl, Trifluormethyl, Pentafluorethyl, Pentachlorethyl, Fluortetrachlorethyl, Difluortrichlorethyl, Trifluordichlorethyl, Tetrafluorchlorethyl, Heptafluorpropyl, Chlorethyl, Bromethyl, Chlorpropyl, Brompropyl, Dichlormethyl, Chlorfluormethyl, Trichlormethyl, Trifluorethyl, Trifluorchlorethyl, Tetrafluorethyl, Difluorchlorethyl, Fluordibrommethyl, Difluorbrommethyl, Fluorchlorbrommethyl, Chlorallyl, Fluorallyl, Chlorbutenyl, Fluorbutenyl, Dichlorallyl, Fluorchlorallyl, Difluorallyl, Bromallyl, für Methoxymethyl, Methoxyethyl, Methoxypropyl, Ethoxymethyl, Ethoxyethyl, Methylthiomethyl, Methylsulfinylmethyl, Methylsulfonylethyl, Methylthioethyl, Methylthiopropyl, Methylsulfinylethyl, Methylsulfonylmethyl oder für jeweils gegebenenfalls einfach bis dreifach, gleich oder verschieden substituiertes Phenyl, Benzyl oder Phenylethyl steht, wobei als Phenylsubstituenten jeweils in Frage kommen: Fluor, Chlor, Brom, Iod, Cyano, Nitro, Methyl, Ethyl, Methoxy, Methylthio, Trifluormethyl, Methylsulfinyl, Methylsulfonyl, Trifluormethoxy, Trifluormethylthio, Trifluormethylsulfinyl oder Trifluormethylsulfonyl,

n für eine Zahl 0, 1 oder 2 steht,

$R^3$ für Cyano, für einen Rest

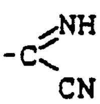

oder für einen Rest $-\underset{\underset{O}{\|}}{C}-Y-R^4$ steht und

Ar für ein-bis fünffach, gleich oder verschieden substituiertes Phenyl oder für jeweils gegebenenfalls ein-bis vierfach gleich oder verschieden substituiertes 2-Pyridyl oder 4-Pyridyl steht, wobei als Phenyl-bzw. Pyridylsubstituenten jeweils in Frage kommen: Cyano, Nitro, Fluor, Chlor, Brom, Iod, Methyl, Ethyl, n-und i-Propyl, n-, i-, s-und t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder einen Rest $-S(O)_p-R^8$,

wobei

Y für Sauerstoff, Schwefel oder einen Rest $-\underset{|}{N}-R^5$ steht,

$R^4$ für Wasserstoff, für gegebenenfalls ein-bis dreifach, gleich oder verschieden durch Fluor, Chlor, Hydroxy, Methoxy, Ethoxy, Methylthio oder Ethylthio substituiertes Methyl, Ethyl, n-oder i-Propyl, n-, i-, s-

oder t-Butyl, n-oder i-Pentyl, für gegebenenfalls ein-bis dreifach, gleich oder verschieden durch Fluor oder Chlor substituiertes Allyl, Propenyl oder Butenyl, für Propargyl, Propinyl, Butinyl oder Pentinyl, für Cyclopropyl, Cyclopentyl, Cyclohexyl, für jeweils gegebenenfalls ein-bis fünffach, gleich oder verschieden durch Fluor, Chlor, Brom, Cyano, Nitro, Methyl, Ethyl, Methoxy, Ethoxy, Methylthio oder Trifluormethyl substituiertes Benzyl oder Phenyl steht, für einen Rest $-A-\overset{\overset{O}{\|}}{C}-Z-R^6$ steht,

und außerdem für den Fall, daß Y für Sauerstoff, Schwefel oder einen Rest
$-\underset{\underset{SO_2-R^7}{|}}{N}-$ steht,
auch für ein Äquivalent eines Natrium-, Kalium-, Magnesium-, Calcium-, Barium-, Kupfer-, Zink-, Mangan-, Zinn-, Eisen-, Cobalt-oder Nickel kations oder für ein gegebenenfalls ein-bis dreifach, gleich oder verschieden durch Methyl, Ethyl, n-oder i-Propyl, n-, i-, s-oder t-Butyl, Benzyl oder Phenyl substituiertes Ammonium-, Phosphonium-oder Sulfoniumkation steht,

$R^5$ für Wasserstoff, für gegebenenfalls ein-bis dreifach, gleich oder verschieden durch Fluor, Chlor, Hydroxy, Methoxy, Ethoxy, Methylthio oder Ethylthio substituiertes Methyl, Ethyl, n-oder i-Propyl, n-, i-, s-oder t-Butyl, n-oder i-Pentyl, für gegebenenfalls ein-bis dreifach, gleich oder verschieden durch Fluor oder Chlor substituiertes Allyl, Propenyl oder Butenyl, für Propargyl, Propinyl, Butinyl oder Pentinyl, für Cyclopropyl, Cyclopentyl, Cyclohexyl, für jeweils gegebenenfalls ein-bis fünffach, gleich oder verschieden durch Fluor, Chlor, Brom, Cyano, Nitro, Methyl, Ethyl, Methoxy, Ethoxy, Methylthio oder Trifluormethyl substituiertes Benzyl oder Phenyl und außerdem für einen Rest $-SO_2-R^7$ steht,

A für einen zweifach verknüpften geradkettigen oder verzweigten Alkylenrest mit 1 bis 6 Kohlenstoffatomen steht,

Z für Sauerstoff, Schwefel, einen N-Methyl-oder einen N-Ethylrest steht,

$R^6$ Wasserstoff, Methyl, Ethyl, n-oder i-Propyl, n-, i-, s-oder t-Butyl, für Allyl, Propenyl, Butenyl, Propargyl, Propinyl oder Butinyl steht,

$R^7$ für Methyl, Ethyl, n-oder i-Propyl, n-, i-, s-oder t-Butyl oder für gegebenenfalls ein-bis fünffach, gleich oder verschieden durch Fluor, Chlor, Brom, Cyano, Nitro, Methyl, Ethyl, Methoxy, Methylthio oder Trifluormethyl substituiertes Benzyl oder Phenyl steht,

$R^8$ für Amino, Methylamino, Ethylamino, Dimethylamino, Diethylamino, Fluordichlormethyl, Difluorchlormethyl, Tetrafluorethyl, Trifluorchlorethyl, Trifluormethyl, Methyl oder Ethyl steht und

p für eine Zahl 0, 1 oder 2 steht.

Ganz besonders bevorzugt sind Verbindungen der Formel (I), bei welchen

$R^1$ für Wasserstoff, Methyl, t-Butyl oder Trifluormethyl steht,

$R^2$ für Methyl, Ethyl, Trifluormethyl, Dichlorfluormethyl oder Difluorchlormethyl steht,

n für eine Zahl 0, 1 oder 2 steht,

$R^3$ für Cyano, für einen Rest

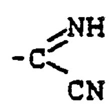

oder für einen Rest $-\overset{\overset{O}{\|}}{C}-Y-R^4$ steht und

Ar für ein-bis fünffach, gleich oder verschieden substituiertes Phenyl oder für jeweils gegebenenfalls ein-bis vierfach gleich oder verschieden substituiertes 2-Pyridyl oder 4-Pyridyl steht, wobei als Phenyl-bzw. Pyridylsubstituenten jeweils in Frage kommen: Cyano, Nitro, Fluor, Chlor, Brom, Iod, Methyl, Ethyl, n-und i-Propyl, n-, i-, s-und t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder einen Rest $-S(O)_p-R^8$,

wobei

Y für Sauerstoff, Schwefel oder für einen Rest $-\underset{|}{N}-R^5$ steht,

$R^4$ für Methyl, Ethyl, n-oder i-Propyl, n-, i-, s-oder t-Butyl, n-oder i-Pentyl, Allyl, Propenyl, n-oder i-Butenyl, n-oder i-Pentenyl, Propargyl, Propinyl, n-oder i-Butinyl, n-oder i-Pentinyl, Trifluorethyl, Trichlorethyl, Chlorethyl, Chlorpropenyl, Dichlorpropenyl, Hydroxyethyl, Hydroxypropyl, Methoxymethyl, Methoxyethyl, Methoxypropyl, Ethoxymethyl, Ethoxyethyl, Ethoxypropyl, Propoxymethyl, Propoxyethyl, Butoxymethyl, Butoxyethyl, Methylthiomethyl, Methylthioethyl, Methylthiopropyl, Ethylthioethyl, Ethylthiopropyl oder Propylthioethyl steht, und außerdem für den Fall, daß für einen Rest $\underset{SO_2-R^7}{\underset{|}{-N-}}$ steht, auch für ein Natrium-oder Kaliumion oder für ein gegebenenfalls ein-bis vierfach, gleich oder verschieden durch Methyl, Ethyl, n-oder i-Propyl, n-Butyl oder Benzyl substituiertes Ammoniumion steht,

$R^5$ für Wasserstoff, Methyl, Ethyl, n-oder i-Propyl, n-, i-, s-oder t-Butyl, n-oder i-Pentyl, Allyl, Propenyl, n-oder i-Butenyl, n-oder i-Pentenyl, Propargyl, Propinyl, n-oder i-Butinyl, n-oder i-Pentinyl, Trifluorethyl, Trichlorethyl, Chlorethyl, Chlorpropenyl, Dichlorpropenyl, Hydroxyethyl, Hydroxypropyl, Methoxymethyl, Methoxyethyl, Methoxypropyl, Ethoxymethyl, Ethoxyethyl, Ethoxypropyl, Methylthiomethyl, Methylthioethyl, Methylthiopropyl, Ethylthioethyl oder Ethylthiopropyl steht, oder für einen Rest $-SO_2-R^7$ steht, wobei

$R^7$ für Methyl, Ethyl, p-Tolyl oder Phenyl steht,

$R^8$ für Amino, Methylamino, Ethylamino, Dimethylamino, Diethylamino, Fluordichlormethyl, Difluorchlormethyl, Tetrafluorethyl, Trifluorchlorethyl, Trifluormethyl, Methyl oder Ethyl steht und

p für eine Zahl 0, 1 oder 2 steht.

Ganz besonders bevorzugt sind außerdem Verbindungen der Formel (I), bei welchen

$R^1$ für Wasserstoff, Methyl, t-Butyl oder Trifluormethyl steht,

$R^2$ für Methyl, Ethyl, Trifluormethyl, Dichlorfluormethyl oder Difluorchlormethyl steht,

n für eine Zahl 0, 1 oder 2 steht,

$R^3$ für einen Rest $-\underset{\underset{O}{\|}}{C}-Y-R^4$ steht und

Ar für ein-bis fünffach, gleich oder verschieden substituiertes Phenyl oder für jeweils gegebenenfalls ein-bis vierfach gleich oder verschieden substituiertes 2-Pyridyl oder 4-Pyridyl steht, wobei als Phenyl-bzw. Pyridylsubstituenten jeweils in Frage kommen: Cyano, Nitro, Fluor, Chlor, Brom, Iod, Methyl, Ethyl, n-und i-Propyl, n-, i-, s-und t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder einen Rest $-S(O)_p-R^8$,

wobei

Y für Sauerstoff oder Schwefel steht,

$R^4$ für Wasserstoff oder für ein Äquivalent eines Natrium-, Kalium-, Calcium-Magnesium-, Barium-, Kupfer-, Zink-, Mangan-, Zinn-, Eisen-, Cobalt-oder Nickelkations steht oder für ein gegebenenfalls ein-bis vierfach, gleich oder verschieden durch Methyl, Ethyl, n-oder i-Propyl, n-, i-oder s-Butyl, Benzyl oder Phenyl substituiertes Ammoniumkation steht,

$R^8$ für Amino, Methylamino, Ethylamino, Dimethylamino, Diethylamino, Fluordichlormethyl, Difluorchlormethyl, Tetrafluorethyl, Trifluorchlorethyl, Trifluormethyl, Methyl oder Ethyl steht und

p für eine Zahl 0, 1 oder 2 steht.

Im einzelnen seien außer den bei den Herstellungsbeispielen genannten Verbindungen die folgenden substituierten 1-Arylpyrazole der allgemeinen Formel (I) genannt:

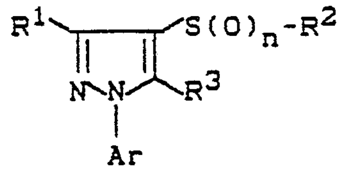

| $R^1$ | $-S(O)_n-R^3$ | $R^3$ | Ar |
|---|---|---|---|
| H | $-SCF_3$ | $-CO-O-CH_2-$ 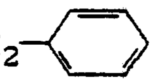 | 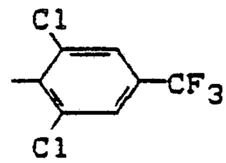 |
| H | $-SCF_3$ | $-CO-O-CH_2-$ 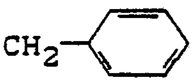 | 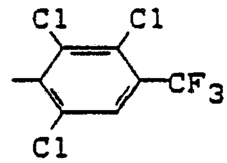 |

| $R^1$ | $-S(O)_n-R^3$ | $R^3$ | Ar |
|---|---|---|---|
| H | $-SCF_3$ | $-CO-O-CH_2-$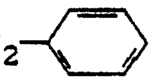 | 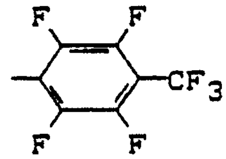 |
| H | $-SCF_3$ | $-CO-O-CH_2-$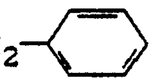 | 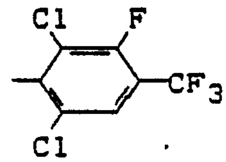 |
| H | $-SCF_3$ | $-CO-O-CH_2-$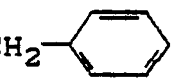 | 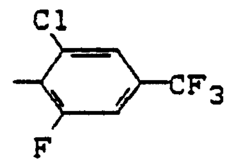 |
| H | $-SCF_3$ | $-CO-O-CH_2-$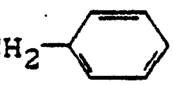 | 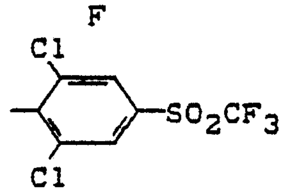 |
| H | $-SCF_3$ | $-CO-O-CH_2-$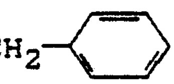 | 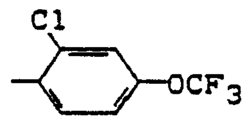 |
| H | $-SCF_3$ | $-CO-O-CH_2-$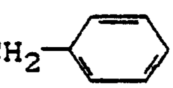 | 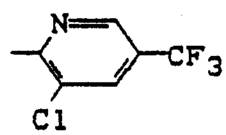 |
| H | $-SCF_3$ | $-CO-O-CH_2-$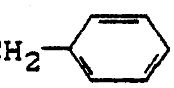 | 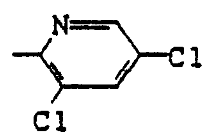 |
| H | $-SCF_3$ | $-CO-O-CH_2-$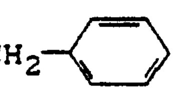 | 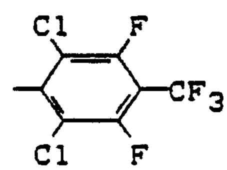 |

| $R^1$ | $-S(O)_n-R^3$ | $R^3$ | Ar |
|---|---|---|---|
| H | $-SCCl_2F$ | -CO-O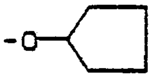 | 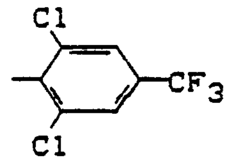 |
| H | $-SCCl_2F$ | -CO-O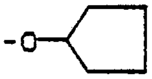 | 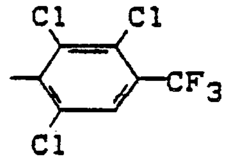 |
| H | $-SCCl_2F$ | -CO-O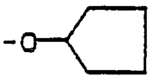 | 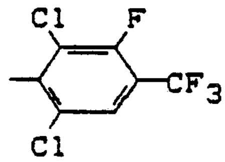 |
| H | $-SCCl_2F$ | -CO-O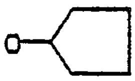 | 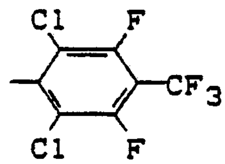 |
| H | $-SCCl_2F$ | -CO-O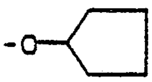 | 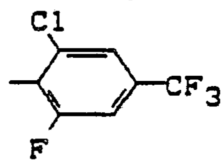 |
| H | $-SCCl_2F$ | -CO-O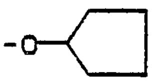 | 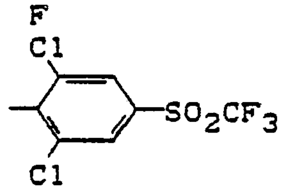 |
| H | $-SCCl_2F$ | -CO-O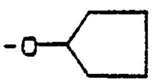 | 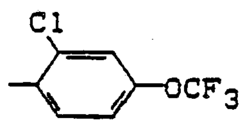 |
| H | $-SCCl_2F$ | -CO-O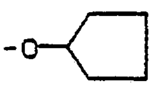 | 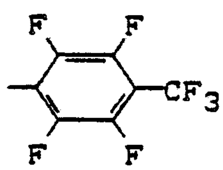 |
| H | $-SCCl_2F$ | -CO-O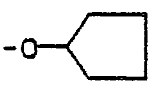 | 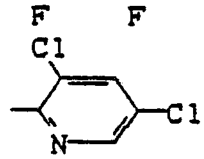 |
| H | $-SCCl_2F$ | -CO-O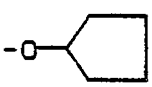 | 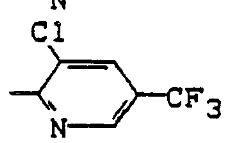 |

| $R^1$ | $-S(O)_n-R^3$ | $R^3$ | Ar |
|---|---|---|---|
| H | $-SO_2-CCl_2F$ | $-CO-O-CH_2CH_2OCH_3$ | 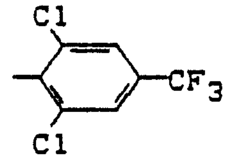 |
| H | $-SO_2-CCl_2F$ | $-CO-O-CH_2CH_2OCH_3$ | 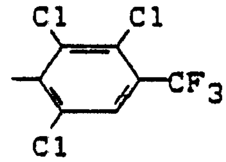 |
| H | $-SO_2-CCl_2F$ | $-CO-O-CH_2CH_2OCH_3$ | 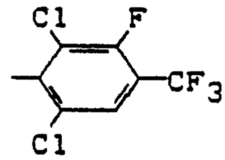 |
| H | $-SO_2-CCl_2F$ | $-CO-O-CH_2CH_2OCH_3$ | 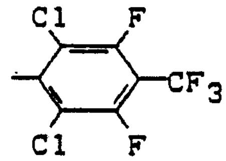 |
| H | $-SO_2-CCl_2F$ | $-CO-O-CH_2CH_2OCH_3$ | 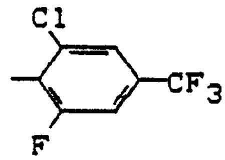 |
| H | $-SO_2-CCl_2F$ | $-CO-O-CH_2CH_2OCH_3$ | 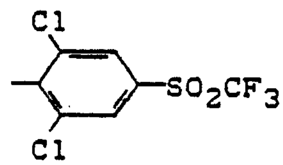 |
| H | $-SO_2-CCl_2F$ | $-CO-O-CH_2CH_2OCH_3$ | 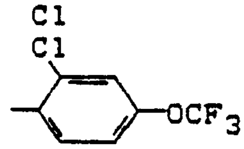 |
| H | $-SO_2-CCl_2F$ | $-CO-O-CH_2CH_2OCH_3$ | 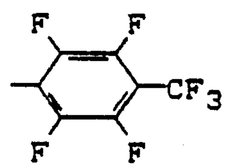 |
| H | $-SO_2-CCl_2F$ | $-CO-O-CH_2CH_2OCH_3$ | 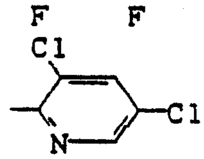 |
| H | $-SO_2-CCl_2F$ | $-CO-O-CH_2CH_2OCH_3$ | 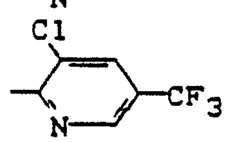 |

| $R^1$ | $-S(O)_n-R^3$ | $R^3$ | Ar |
|---|---|---|---|
| H | $-SO_2-CF_3$ | $-CO-S-CH_3$ | 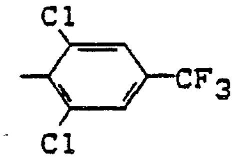 |
| H | $-SO_2-CF_3$ | $-CO-S-CH_3$ | 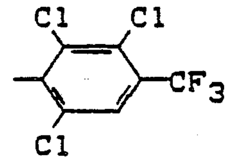 |
| H | $-SO_2-CF_3$ | $-CO-S-CH_3$ | 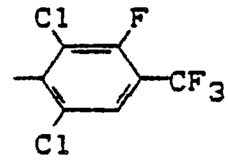 |
| H | $-SO_2-CF_3$ | $-CO-S-CH_3$ | 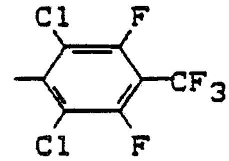 |
| H | $-SO_2-CF_3$ | $-CO-S-CH_3$ | 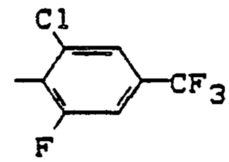 |
| H | $-SO_2-CF_3$ | $-CO-S-CH_3$ | 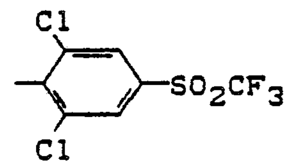 |
| H | $-SO_2-CF_3$ | $-CO-S-CH_3$ | 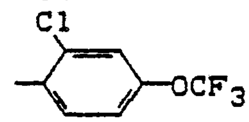 |
| H | $-SO_2-CF_3$ | $-CO-S-CH_3$ | 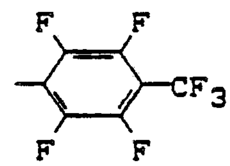 |
| H | $-SO_2-CF_3$ | $-CO-S-CH_3$ | 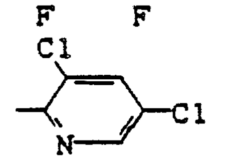 |
| H | $-SO_2-CF_3$ | $-CO-S-CH_3$ | 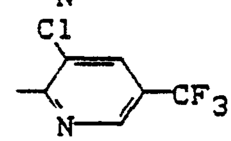 |

| $R^1$ | $-S(O)_n-R^3$ | $R^3$ | Ar |
|---|---|---|---|
| H | $-S-CF_3$ | $-CO-O-CH_2-CH=CH_2$ | 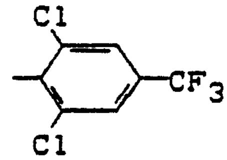 |
| H | $-S-CF_3$ | $-CO-O-CH_2-CH=CH_2$ | 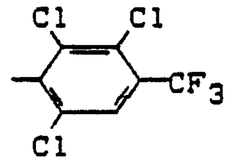 |
| H | $-S-CF_3$ | $-CO-O-CH_2-CH=CH_2$ | 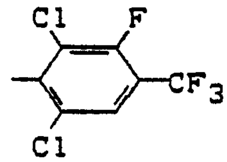 |
| H | $-S-CF_3$ | $-CO-O-CH_2-CH=CH_2$ | 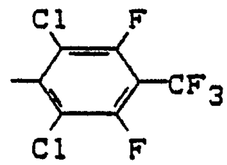 |
| H | $-S-CF_3$ | $-CO-O-CH_2-CH=CH_2$ | 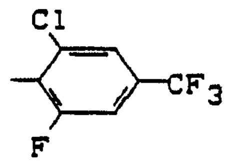 |
| H | $-S-CF_3$ | $-CO-O-CH_2-CH=CH_2$ | 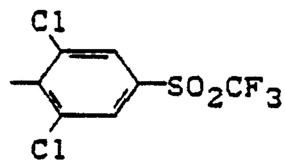 |
| H | $-S-CF_3$ | $-CO-O-CH_2-CH=CH_2$ | 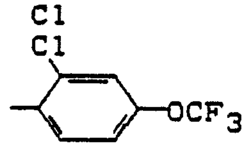 |
| H | $-S-CF_3$ | $-CO-O-CH_2-CH=CH_2$ | 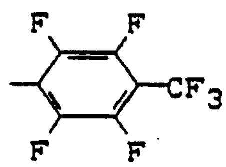 |
| H | $-S-CF_3$ | $-CO-O-CH_2-CH=CH_2$ | 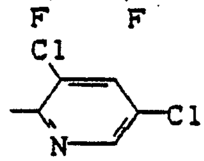 |
| H | $-S-CF_3$ | $-CO-O-CH_2-CH=CH_2$ | 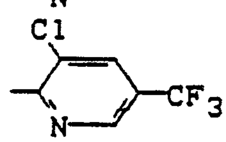 |

| $R^1$ | $-S(O)_n-R^3$ | $R^3$ | Ar |
|---|---|---|---|
| H | $-SO_2-CF_3$ | $-CO-O-CH_2-C\equiv CH$ | 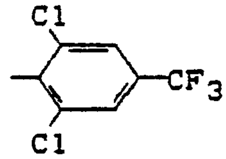 |
| H | $-SO_2-CF_3$ | $-CO-O-CH_2-C\equiv CH$ | 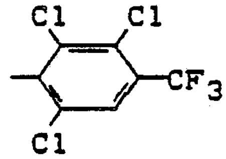 |
| H | $-SO_2-CF_3$ | $-CO-O-CH_2-C\equiv CH$ | 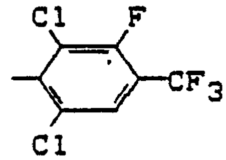 |
| H | $-SO_2-CF_3$ | $-CO-O-CH_2-C\equiv CH$ | 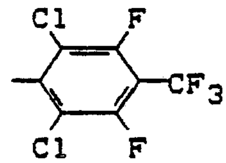 |
| H | $-SO_2-CF_3$ | $-CO-O-CH_2-C\equiv CH$ | 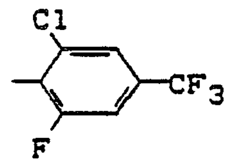 |
| H | $-SO_2-CF_3$ | $-CO-O-CH_2-C\equiv CH$ | 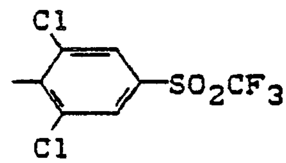 |
| H | $-SO_2-CF_3$ | $-CO-O-CH_2-C\equiv CH$ | 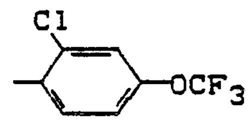 |
| H | $-SO_2-CF_3$ | $-CO-O-CH_2-C\equiv CH$ | 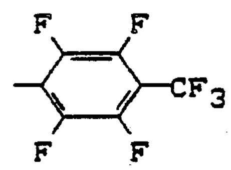 |
| H | $-SO_2-CF_3$ | $-CO-O-CH_2-C\equiv CH$ | 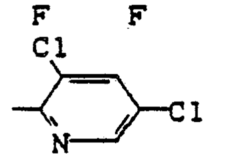 |
| H | $-SO_2-CF_3$ | $-CO-O-CH_2-C\equiv CH$ | 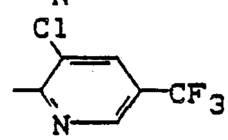 |

| $R^1$ | $-S(O)_n-R^3$ | $R^3$ | Ar |
|---|---|---|---|
| H | $-S-CCl_2F$ | $-CO-O-C(CH_3)_3$ | 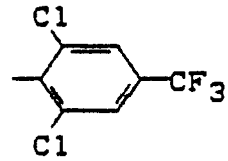 |
| H | $-S-CCl_2F$ | $-CO-O-C(CH_3)_3$ | 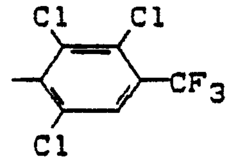 |
| H | $-S-CCl_2F$ | $-CO-O-C(CH_3)_3$ | 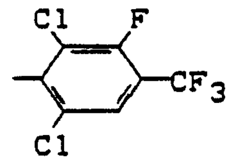 |
| H | $-S-CCl_2F$ | $-CO-O-C(CH_3)_3$ | 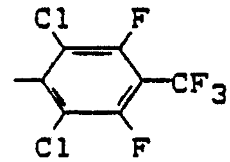 |
| H | $-S-CCl_2F$ | $-CO-O-C(CH_3)_3$ | 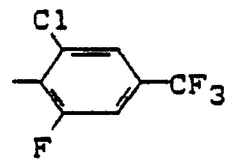 |
| H | $-S-CCl_2F$ | $-CO-O-C(CH_3)_3$ | 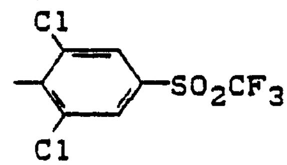 |
| H | $-S-CCl_2F$ | $-CO-O-C(CH_3)_3$ | 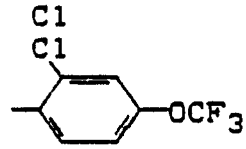 |
| H | $-S-CCl_2F$ | $-CO-O-C(CH_3)_3$ | 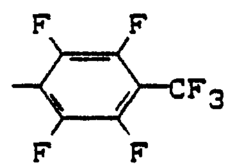 |
| H | $-S-CCl_2F$ | $-CO-O-C(CH_3)_3$ | 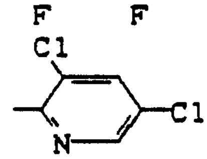 |
| H | $-S-CCl_2F$ | $-CO-O-C(CH_3)_3$ | 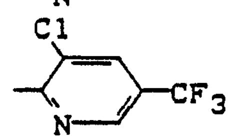 |

| $R^1$ | $-S(O)_n-R^3$ | $R^3$ | Ar |
|---|---|---|---|
| H | $-S-CF_3$ | $-CO-O-CH_2CF_3$ | 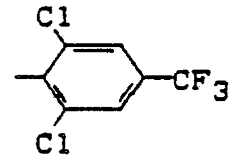 |
| H | $-S-CF_3$ | $-CO-O-CH_2CF_3$ | 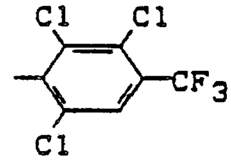 |
| H | $-S-CF_3$ | $-CO-O-CH_2CF_3$ | 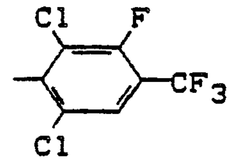 |
| H | $-S-CF_3$ | $-CO-O-CH_2CF_3$ | 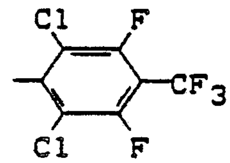 |
| H | $-S-CF_3$ | $-CO-O-CH_2CF_3$ | 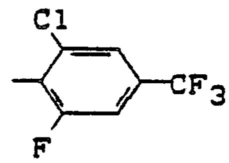 |
| H | $-S-CF_3$ | $-CO-O-CH_2CF_3$ | 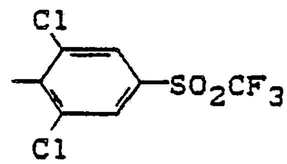 |
| H | $-S-CF_3$ | $-CO-O-CH_2CF_3$ | 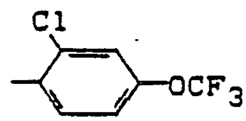 |
| H | $-S-CF_3$ | $-CO-O-CH_2CF_3$ | 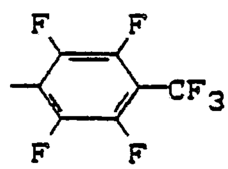 |
| H | $-S-CF_3$ | $-CO-O-CH_2CF_3$ | 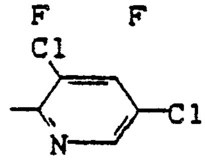 |
| H | $-S-CF_3$ | $-CO-O-CH_2CF_3$ | 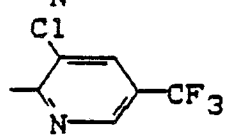 |

| $R^1$ | $-S(O)_n-R^3$ | $R^3$ | Ar |
|---|---|---|---|
| $CH_3$ | $-SCF_3$ | $-CO-O-CH_2-COOC_4H_9-n$ | 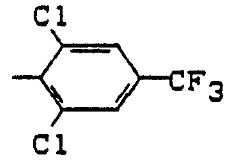 |
| $CH_3$ | $-SCF_3$ | $-CO-O-CH_2-COOC_4H_9-n$ | 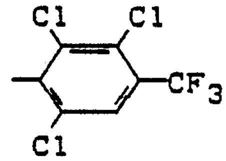 |
| $CH_3$ | $-SCF_3$ | $-CO-O-CH_2-COOC_4H_9-n$ | 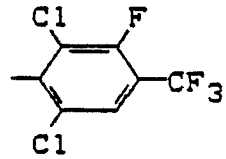 |
| $CH_3$ | $-SCF_3$ | $-CO-O-CH_2-COOC_4H_9-n$ | 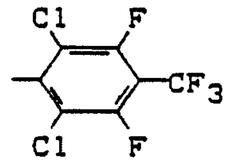 |
| $CH_3$ | $-SCF_3$ | $-CO-O-CH_2-COOC_4H_9-n$ | 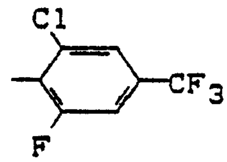 |
| $CH_3$ | $-SCF_3$ | $-CO-O-CH_2-COOC_4H_9-n$ | 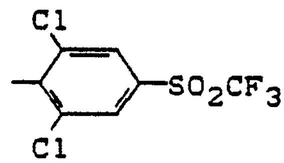 |
| $CH_3$ | $-SCF_3$ | $-CO-O-CH_2-COOC_4H_9-n$ | 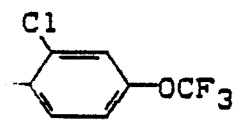 |
| $CH_3$ | $-SCF_3$ | $-CO-O-CH_2-COOC_4H_9-n$ | 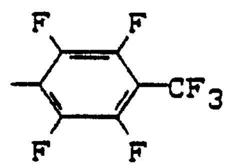 |
| $CH_3$ | $-SCF_3$ | $-CO-O-CH_2-COOC_4H_9-n$ | 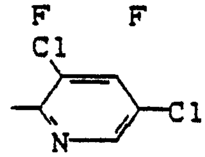 |
| $CH_3$ | $-SCF_3$ | $-CO-O-CH_2-COOC_4H_9-n$ | 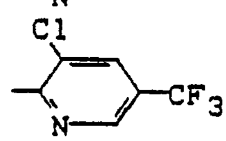 |

| $R^1$ | $-S(O)_n-R^3$ | $R^3$ | Ar |
|---|---|---|---|
| $CH_3$ | $-S-CF_3$ | $CH_3$<br>$-CO-O-CH-COOC_2H_5$ | 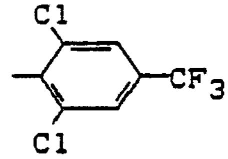 |
| $CH_3$ | $-S-CF_3$ | $CH_3$<br>$-CO-O-CH-COOC_2H_5$ | 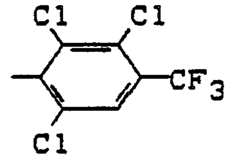 |
| $CH_3$ | $-S-CF_3$ | $CH_3$<br>$-CO-O-CH-COOC_2H_5$ | 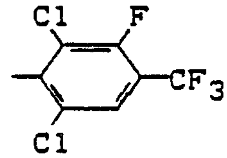 |
| $CH_3$ | $-S-CF_3$ | $CH_3$<br>$-CO-O-CH-COOC_2H_5$ | 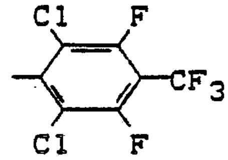 |
| $CH_3$ | $-S-CF_3$ | $CH_3$<br>$-CO-O-CH-COOC_2H_5$ | 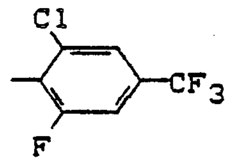 |
| $CH_3$ | $-S-CF_3$ | $CH_3$<br>$-CO-O-CH-COOC_2H_5$ | 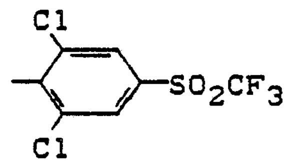 |
| $CH_3$ | $-S-CF_3$ | $CH_3$<br>$-CO-O-CH-COOC_2H_5$ | 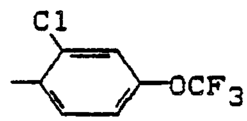 |
| $CH_3$ | $-S-CF_3$ | $CH_3$<br>$-CO-O-CH-COOC_2H_5$ | 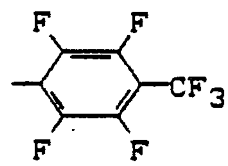 |
| $CH_3$ | $-S-CF_3$ | $CH_3$<br>$-CO-O-CH-COOC_2H_5$ | 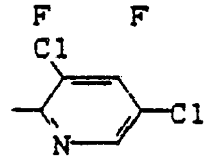 |
| $CH_3$ | $-S-CF_3$ | $CH_3$<br>$-CO-O-CH-COOC_2H_5$ | 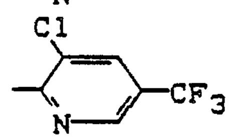 |

| $R^1$ | $-S(O)_n-R^3$ | $R^3$ | Ar |
|---|---|---|---|
| $CH_3$ | $-SO_2-CF_3$ | $-CO-N(C_2H_5)_2$ | 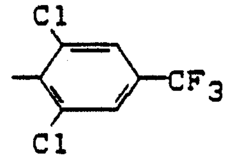 |
| $CH_3$ | $-SO_2-CF_3$ | $-CO-N(C_2H_5)_2$ | 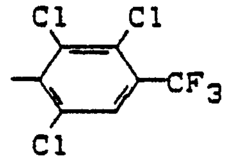 |
| $CH_3$ | $-SO_2-CF_3$ | $-CO-N(C_2H_5)_2$ | 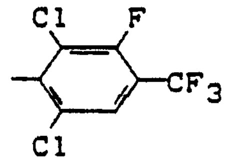 |
| $CH_3$ | $-SO_2-CF_3$ | $-CO-N(C_2H_5)_2$ | 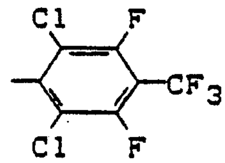 |
| $CH_3$ | $-SO_2-CF_3$ | $-CO-N(C_2H_5)_2$ | 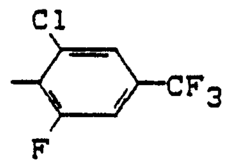 |
| $CH_3$ | $-SO_2-CF_3$ | $-CO-N(C_2H_5)_2$ | 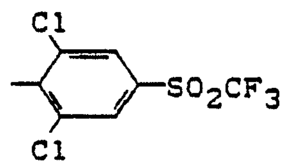 |
| $CH_3$ | $-SO_2-CF_3$ | $-CO-N(C_2H_5)_2$ | 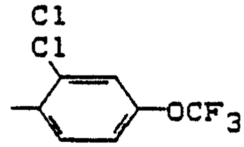 |
| $CH_3$ | $-SO_2-CF_3$ | $-CO-N(C_2H_5)_2$ | 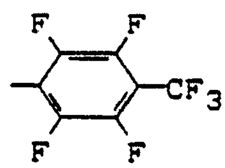 |
| $CH_3$ | $-SO_2-CF_3$ | $-CO-N(C_2H_5)_2$ | 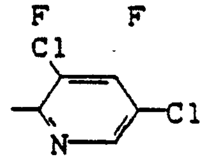 |
| $CH_3$ | $-SO_2-CF_3$ | $-CO-N(C_2H_5)_2$ | 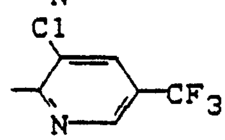 |

| $R^1$ | $-S(O)_n-R^3$ | $R^3$ | Ar |
|---|---|---|---|
| $CH_3$ | $-S-CF_3$ | $-CO-NH-CH_3$ | 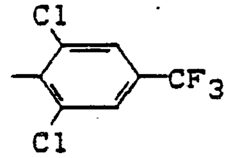 |
| $CH_3$ | $-S-CF_3$ | $-CO-NH-CH_3$ | 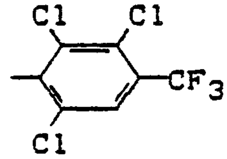 |
| $CH_3$ | $-S-CF_3$ | $-CO-NH-CH_3$ | 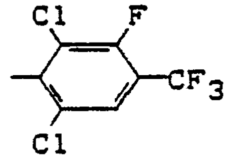 |
| $CH_3$ | $-S-CF_3$ | $-CO-NH-CH_3$ | 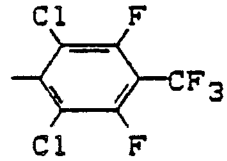 |
| $CH_3$ | $-S-CF_3$ | $-CO-NH-CH_3$ | 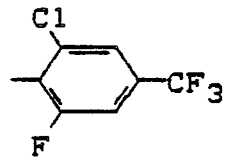 |
| $CH_3$ | $-S-CF_3$ | $-CO-NH-CH_3$ | 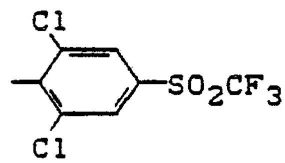 |
| $CH_3$ | $-S-CF_3$ | $-CO-NH-CH_3$ | 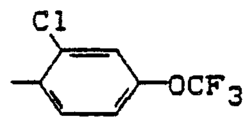 |
| $CH_3$ | $-S-CF_3$ | $-CO-NH-CH_3$ | 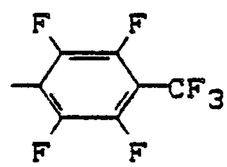 |
| $CH_3$ | $-S-CF_3$ | $-CO-NH-CH_3$ | 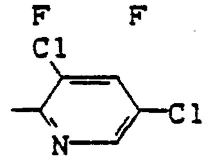 |
| $CH_3$ | $-S-CF_3$ | $-CO-NH-CH_3$ | 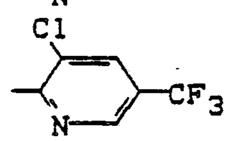 |

| $R^1$ | $-S(O)_n-R^3$ | $R^3$ | Ar |
|---|---|---|---|
| H | $-SO_2-CF_3$ | 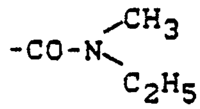 | 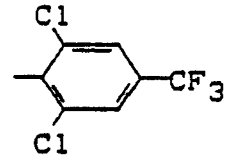 |
| H | $-SO_2-CF_3$ | 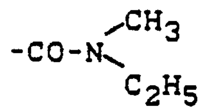 | 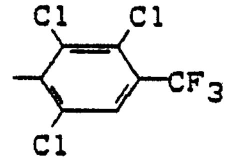 |
| H | $-SO_2-CF_3$ | 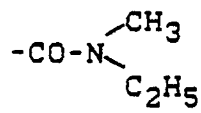 | 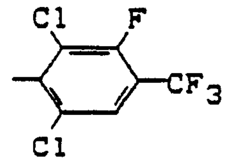 |
| H | $-SO_2-CF_3$ | 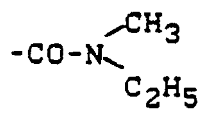 | 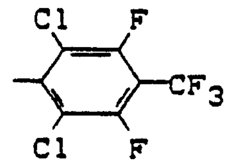 |
| H | $-SO_2-CF_3$ | 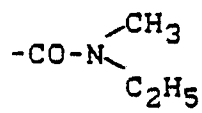 | 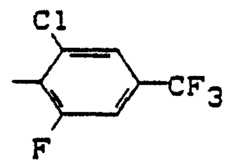 |
| H | $-SO_2-CF_3$ | 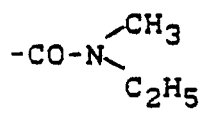 | 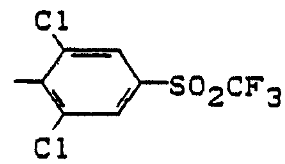 |
| H | $-SO_2-CF_3$ | 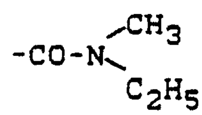 | 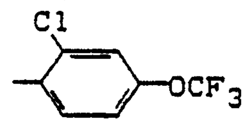 |
| H | $-SO_2-CF_3$ | 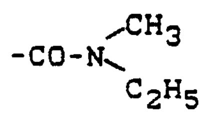 | 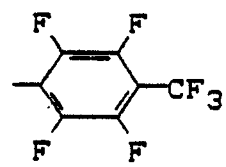 |
| H | $-SO_2-CF_3$ | 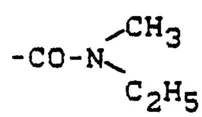 | 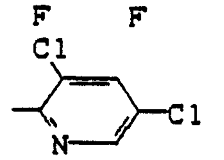 |
| H | $-SO_2-CF_3$ | 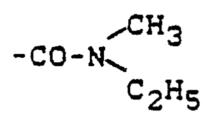 | 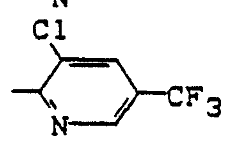 |

| $R^1$ | $-S(O)_n-R^3$ | $R^3$ | Ar |
|---|---|---|---|
| H | $-S-CF_3$ | $-CO-NH-SO_2-CH_3$ | 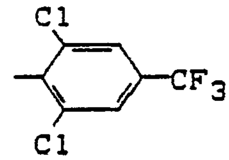 |
| H | $-S-CF_3$ | $-CO-NH-SO_2-CH_3$ | 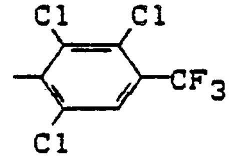 |
| H | $-S-CF_3$ | $-CO-NH-SO_2-CH_3$ | 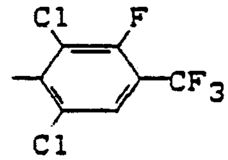 |
| H | $-S-CF_3$ | $-CO-NH-SO_2-CH_3$ | 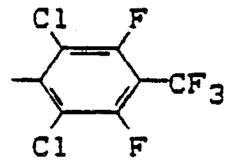 |
| H | $-S-CF_3$ | $-CO-NH-SO_2-CH_3$ | 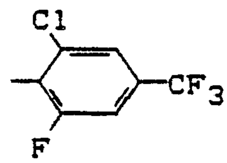 |
| H | $-S-CF_3$ | $-CO-NH-SO_2-CH_3$ | 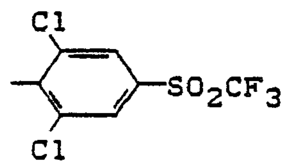 |
| H | $-S-CF_3$ | $-CO-NH-SO_2-CH_3$ | 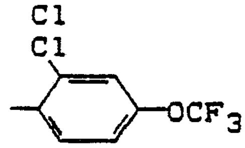 |
| H | $-S-CF_3$ | $-CO-NH-SO_2-CH_3$ | 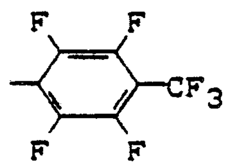 |
| H | $-S-CF_3$ | $-CO-NH-SO_2-CH_3$ | 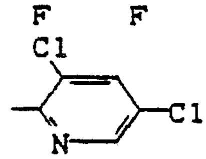 |
| H | $-S-CF_3$ | $-CO-NH-SO_2-CH_3$ | 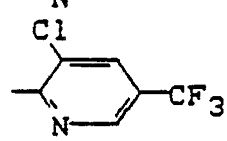 |

| $R^1$ | $-S(O)_n-R^3$ | $R^3$ | Ar |
|---|---|---|---|
| $CH_3$ | $-S-CCl_2F$ | $-COO^{\ominus}\ Na^{\oplus}$ | 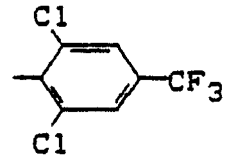 |
| $CH_3$ | $-S-CCl_2F$ | $-COO^{\ominus}\ Na^{\oplus}$ | 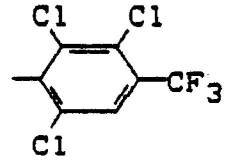 |
| $CH_3$ | $-SO_2-CCl_2F$ | $-COO^{\ominus}\ H_3\overset{\oplus}{N}-C_3H_7$ | 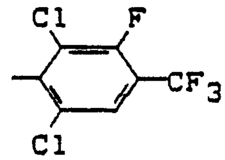 |
| $CH_3$ | $-SO_2-CCl_2F$ | $-COO^{\ominus}\ H_3\overset{\oplus}{N}-C_3H_7$ | 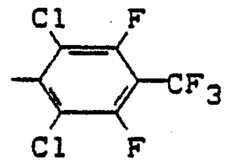 |
| H | $-S-CClF_2$ | $-COOH$ | 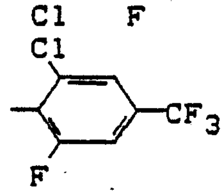 |
| H | $-S-CClF_2$ | $-COOH$ | 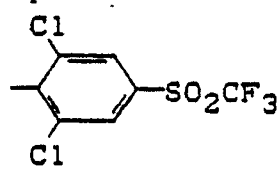 |
| $CH_3$ | $-SO_2-CClF_2$ | $-COOH$ | 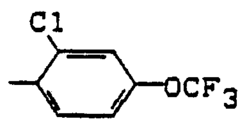 |
| $CH_3$ | $-SO_2-CClF_2$ | $-COOH$ | 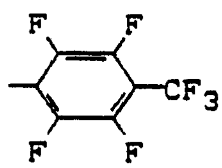 |
| H | $-SO-CF_3$ | $-COOH$ | 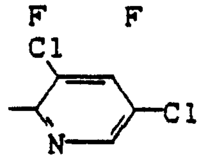 |
| H | $-SO-CF_3$ | $-COOH$ | 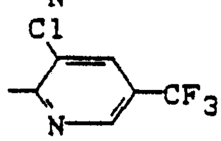 |

Verwendet man beispielsweise 5-Brom-1-(2,6-dichlor-4-trifluormethylphenyl)-4-trifluormethylthio-pyrazol und Kohlendioxid als Ausgangsstoffe, so läßt sich der Reaktionsablauf des erfindungsgemäßen Verfahrens (a) durch das folgende Formelschema darstellen:

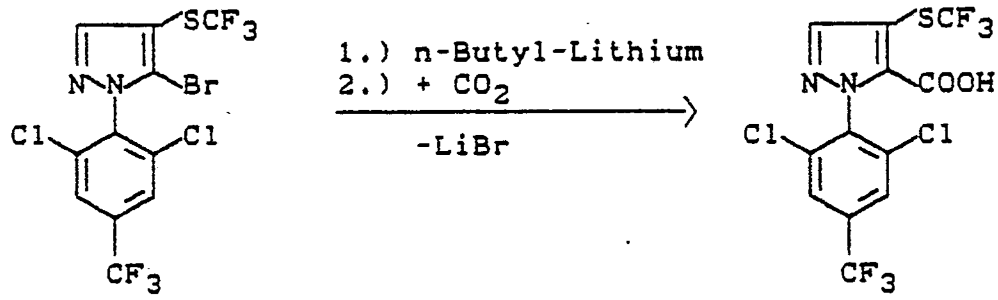


Verwendet man beispielsweise 5-Carboxy-4-dichlorfluormethylsulfenyl-1-(2,4,6-trichlorphenyl)-pyrazol und Methanol als Ausgangsstoffe sowie Thionylchlorid als Reaktionshilfsmittel, so läßt sich der Reaktionsablauf des erfindungsgemäßen Verfahrens (b) durch das folgende Formelschema darstellen:

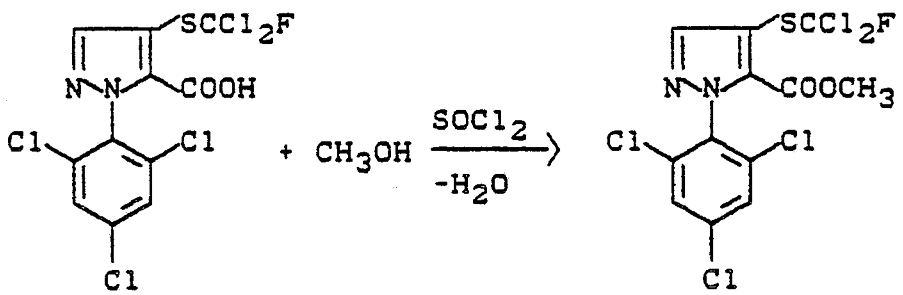


Verwendet man beispielsweise 4-Dichlorfluormethylsulfonyl-1-(3,5-dichlor-2-pyridyl)-pyrazol-5-yl-thiocarbonsäure und Natriumhydroxid als Ausgangsstoffe, so läßt sich der Reaktionsablauf des erfindungsgemäßen Verfahrens (c) durch das folgende Formelschema darstellen:

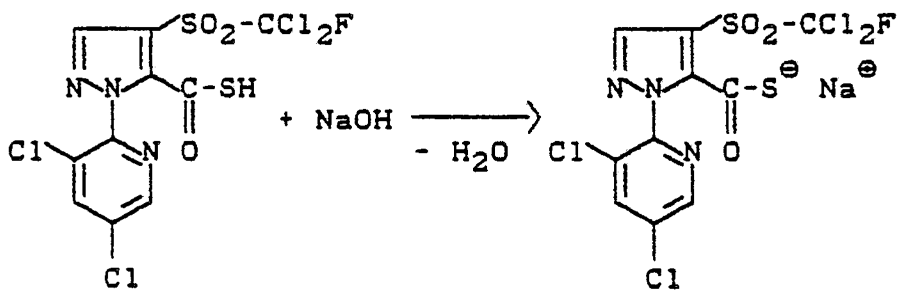


Verwendet man beispielsweise 3-Methyl-4-trifluormethylsulfenyl-1-(2,3,6-trichlor-4-trifluormethylphenyl)-pyrazol-5-yl-carbonsäure Kaliumsalz und 2-Brompropionsäuremethylester als Ausgangsstoffe, so läßt sich der Reaktionsablauf des erfindungsgemäßen Verfahrens (d) durch das folgende Formelschema darstellen:

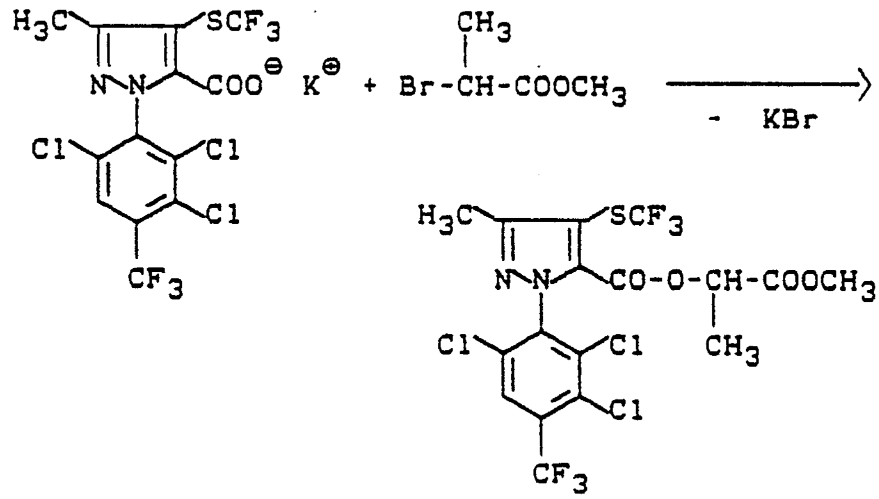


Verwendet man beispielsweise 5-Brom-4-trifluormethylsulfenyl-1-(2,3,5,6-tetrafluor-4-trifluormethyl-phenyl)-pyrazol und Dicyan als Ausgangsstoffe, so läßt sich der Reaktionsablauf des erfindungsgemäßen Verfahrens (e) durch das folgende Formelschema darstellen:

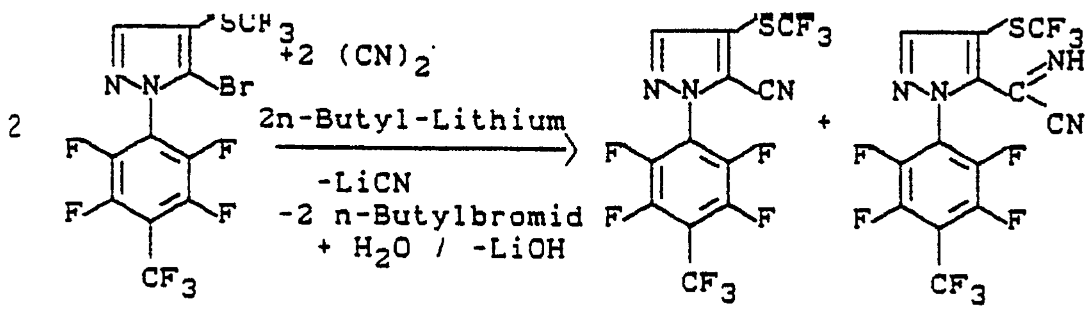


Verwendet man beispielsweise 5-Cyano-4-trifluormethylsulfenyl-1-(2,6-dichlor-4-trifluormethylphenyl)-pyrazol als Ausgangsverbindung und m-Chlorperbenzoesäure als Oxidationsmittel, so läßt sich der Reaktionsablauf des erfindungsgemäßen Verfahrens (f) durch das folgende Formelschema darstellen:

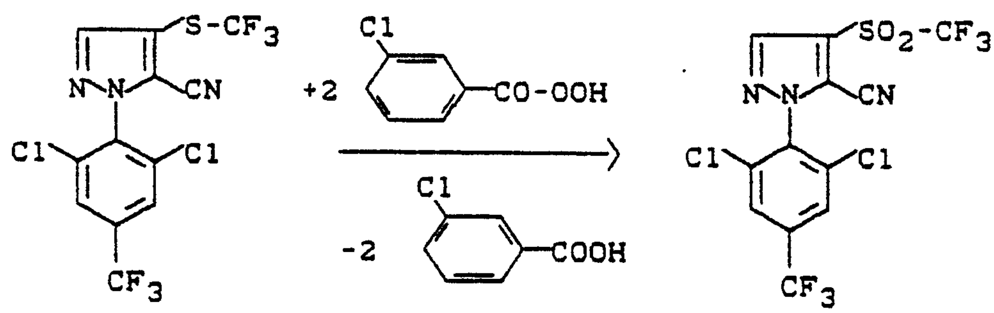


Die zur Durchführung der erfindungsgemäßen Verfahrens (a) und (e) als Ausgangsstoffe benötigten 5-Halogen-1-aryl-pyrazole sind durch die Formel (II) allgemein definiert. In dieser Formel (II) stehen $R^1$, $R^2$, Ar und n vorzugsweise für diejenigen Reste, die bereits im Zusammenhang mit der Beschreibung der erfindungsgemäßen Stoffe der Formel (I) als bevorzugt für diese Substituenten genannt wurden. Hal steht vorzugsweise für Chlor oder Brom.

Die 5-Halogen-1-aryl-pyrazole der Formel (II) sind bekannt (vgl. DE-OS 35 29 829).

Die zur Durchführung des erfindungsgemäßen Verfahrens (b) als Ausgangsstoffe benötigten substituierten 1-Arylpyrazole sind durch die Formel (Ia) allgemein definiert. In dieser Formel (Ia) stehen $R^1$, $R^2$, Ar und n vorzugsweise für diejenigen Reste, die bereits im Zusammenhang mit der Beschreibung der erfindungsgemäßen Stoffe der Formel (I) als bevorzugt für diese Substituenten genannt wurden.

Die substituierten 1-Arylpyrazole der Formel (Ia) sind erfindungsgemäße Verbindungen und erhältlich mit Hilfe des erfindunsggemäßen Verfahrens (a).

Die zur Durchführung des erfindungsgemäßen Verfahrens (b) weiterhin als Ausgangsstoffe benötigten Alkohole, Amine und Thiole sind durch die Formel (II) allgemein definiert. In dieser Formel (III) steht Y vorzugsweise für diejenigen Reste, die bereits im Zusammenhang mit der Beschreibung der erfindungsgemäßen Stoffe der Formel (I) als bevorzugt für diese Substituenten genannt wurden.

$R^{4-1}$ steht vorzugsweise für jeweils geradkettiges oder verzweigtes Alkyl, Hydroxyalkyl, Alkoxyalkyl, Alkylthioalkyl, Halogenalkyl, Alkenyl, Halogenalkenyl oder Alkinyl mit jeweils bis zu 12 Kohlenstoffatomen und im Fall des Halogenalkyl bzw. Halogenalkenyl mit 1 bis 15 gleichen oder verschiedenen Halogenatomen, für Cycloalkyl mit 3 bis 7 Kohlenstoffatomen oder für jeweils gegebenenfalls einfach oder mehrfach, gleich oder verschieden durch Halogen, Cyano, Nitro, jeweils geradkettiges oder verzweigtes Alkyl, Alkoxy, Alkylthio oder Halogenalkyl mit jeweils 1 bis 4 Kohlenstoffatomen und im Fall des Halogenalkyls mit 1 bis 9 gleichen oder verschiedenen Halogenatomen substituiertes Phenyl, Benzyl oder Phenethyl; außerdem für einen Rest $-A-\underset{\underset{O}{\|}}{C}-Z-R^6$, wobei

A für einen zweifach verknüpften geradkettigen oder verzweigten Alkylenrest mit 1 bis 8 Kohlenstoffatomen steht,

Z für Sauerstoff, Schwefel oder einen N-Alkylrest mit 1 bis 4 Kohlenstoffatomen im geradkettigen oder verzweigten Alkylteil steht und

$R^6$ für Wasserstoff oder für jeweils geradkettiges oder verzweigtes Alkyl, Alkenyl oder Alkinyl mit jeweils bis zu 6 Kohlenstoffatomen steht,

$R^{4-1}$ steht darüberhinaus für den Fall, daß Y für Schwefel oder für einen Rest $-\underset{|}{N}-R^5$ steht, wobei $R^5$ vorzugsweise für diejenigen Substituenten steht, die bereits im Zusammenhang mit der Beschreibung der erfindungsgemäßen Stoffe der Formel (I) als bevorzugt für diesen Rest genannt wurden, vorzugsweise auch für Wasserstoff.

Die Alkohole, Amine oder Thiole der Formel (III) sind allgemein bekannte Verbindungen der organischen Chemie.

Die zur Durchführung des erfindungsgemäßen Verfahrens (c) als Ausgangsstoffe benötigten substituierte 1-Arylpyrazole sind durch die Formel (Ia1) allgemein definiert. In dieser Formel (Ia1) stehen $R^1$, $R^2$, Ar und n vorzugsweise für diejenigen Reste und Indices, die bereits im Zusammenhang mit der Beschreibung der erfindungsgemäßen Stoffe der Formel (I) als bevorzugt für diese Substituenten und Indices genannt wurden.

$Y^1$ steht vorzugsweise für Sauerstoff, Schwefel oder einen Rest $-\underset{\underset{SO_2-R^7}{|}}{N}-$, wobei

$R^7$ vorzugsweise für jeweils geradkettiges oer verzweigtes Alkyl oder Halogenalkyl mit jeweils 1 bis 6 Kohlenstoffatomen und im Fall des Halogenalkyls mit 1 bis 9 gleichen oder verschiedenen Halogenatomen oder für gegebenenfalls einfach oder mehrfach, gleich oder verschieden substituiertes Phenyl, Benzyl oder Phenethyl steht, wobei als Substituenten jeweils in Frage kommen: Halogen, Cyano, Nitro, jeweils geradkettiges oder verzweigtes Alkyl, Alkoxy, Alkylthio oder Halogenalkyl mit jeweils 1 bis 4 Kohlenstoffatomen und im Fall des Halogenalkyl mit 1 bis 9 gleichen oder verschiedenen Halogenatomen.

Die substituierten 1-Arylpyrazole der Formel (Ia1) sind erfindungsgemäße Verbindungen und erhältlich mit Hilfe der erfindungsgemäßen Verfahren (a) oder (b).

Die zur Durchführung des erfindungsgemäßen Verfahrens (d) als Ausgangsstoffe benötigten substituierten 1-Arylpyrazole sind durch die Formel (Ic) allgemein definiert. In dieser Formel (Ic) stehen $R^1$, $R^2$, Ar und n vorzugsweise für diejenigen Reste, die bereits im Zusammenhang mit der Beschreibung der erfindungsgemäßen Stoffe der Formel (I) als bevorzugt für diese Substituenten und Indices genannt wurden.

$Y^1$ steht vorzugsweise für diejenigen Reste, die bereits im Zusammenhang mit der Beschreibung der Vorprodukte der Formel (Ia1) als bevorzugt für diesen Substituenten genannt wurden und

$M^{\oplus}$ steht vorzugsweise für ein Äquivalent eines Natrium-, Kalium-, Magnesium-, Calcium-, Barium-, Kupfer-, Zink-, Mangan-, Zinn-, Eisen-, Cobalt-und Nickelkations oder für ein gegebenenfalls ein-bis dreifach gleich oder verschieden durch Methyl, Ethyl, n-oder i-Propyl, n-, i-, s-oder t-Butyl, Benzyl oder Phenyl substituiertes Ammonium-, Phosphonium-oder Sulfoniumkation.

Die substituierten 1-Arylpyrazole der Formel (Ic) sind erfindungsgemäße Verbindungen und erhältlich mit Hilfe des erfindungsgemäßen Verfahrens (c).

Die zur Durchführung des erfindungsgemäßen Verfahrens (d) als Ausgangsstoffe benötigten Alkylierungsmittel sind durch die Formel (IV) allgemein definiert. In dieser Formel (IV) steht

$R^{4-2}$ vorzugsweise für jeweils geradkettiges oder verzweigtes Alkyl, Hydroxyalkyl, Alkoxyalkyl, Alkylthioalkyl, Halogenalkyl, Alkenyl, Halogenalkenyl oder Alkinyl mit jeweils bis zu 12 Kohlenstoffatomen und im Falle des Halogenalkyl bzw. des Halogenalkenyl mit 1 bis 15 gleichen oder verschiedenen Halogenatomen, für Cycloalkyl mit 3 bis 7 Kohlenstoffatomen oder für jeweils gegebenenfalls einfach oder mehrfach, gleich oder verschieden durch Halogen, Cyano, Nitro sowie jeweils geradkettiges oder verzweigtes Alkyl, Alkoxy, Alkylthio oder Halogenalkyl mit jeweils 1 bis 4 Kohlenstoffatomen und im Fall des Halogenalkyls mit 1 bis 9 gleichen oder verschiedenen Halogenatomen substituiertes Phenyl, Benzyl oder Phenethyl,
außerdem für einen Rest $-A-\underset{\underset{O}{\|}}{C}-Z-R^{6}$,

wobei
A für einen zweifach verknüpften geradkettigen oder verzweigten Alkylrest mit 1 bis 8 Kohlenstoffatomen steht,
Z für Sauerstoff, Schwefel oder einen N-Alkylrest mit 1 bis 4 Kohlenstoffatomen im geradkettigen oder verzweigten Alkylteil steht und
$R^6$ für Wasserstoff oder für jeweils geradkettiges oder verzweigtes Alkyl, Alkenyl oder Alkinyl mit jeweils bis zu 6 Kohlenstoffatomen steht.

E steht vorzugsweise für Halogen oder für jeweils gegebenenfalls durch Halogen und/oder geradkettiges oder verzweigtes Alkyl mit 1 bis 4 Kohlenstoffatomen, einfach oder mehrfach, gleich oder verschieden substituiertes Alkylsulfonyloxy, Alkoxysulfonyloxy oder Arylsulfonyloxy, E steht insbesondere für Chlor, Brom, Iod, Methansulfonyloxy, Methoxysulfonyloxy oder p-Toluolsulfonyloxy.

Die Alkylierungsmittel der Formel (IV) sind allgemein bekannte Verbindungen der organischen Chemie.

Die zur Durchführung des erfindungsgemäßen Verfahrens (f) als Ausgangsstoffe benötigten substituierten 1-Arylpyrazole sind durch die Formel (Ig) allgemein definiert. In dieser Formel (Ig) stehen $R^1$, $R^2$, $R^3$ und Ar vorzugsweise für diejenigen Reste, die bereits im Zusammenhang mit der Beschreibung der erfindungsgemäßen Stoffe der Formel (I) als bevorzugt für diese Substituenten genannt wurden.

Die substituierten 1-Arylpyrazole der Formel (Ig) sind erfindungsgemäße Verbindungen und erhältlich mit Hilfe der erfindungsgemäßen Verfahren (a), (b), (c), (d) oder (e).

Als Verdünnungsmittel zur Durchführung der erfindungsgemäßen Verfahren (a) und (e) kommen inerte organische Lösungsmittel in Frage. Hierzu gehören insbesondere aliphatische oder aromatische Kohlenwasserstoffe, wie beispielsweise Benzin, Benzol, Toluol, Xylol, Petrolether, Hexan, Cyclohexan oder Ether wie Diethylether, Dioxan, Tetrahydrofuran oder Ethylenglykoldimethyl-oder -diethylether.

Die erfindungsgemäßen Verfahren (a) und (e) werden in Gegenwart einer geeigneten Lithium-organischen-Verbindung durchgeführt. Als solche kommen alle üblicherweise für derartige Metallierungsreaktionen verwendeten Lithium-organischen-Verbindungen wie beispielsweise Methyllithium, Butyllithium oder Phenyllithium in Frage. Vorzugsweise verwendet man n-Butyllithium.

Die Reaktionstemperaturen können bei der Durchführung der erfindungsgemäßen Verfahren (a) und (e) in einem gewissen Bereich variiert werden. Im allgemeinen arbeitet man bei Temperaturen zwischen -100°C und +50°C, vorzugsweise bei Temperaturen zwischen -80°C und +20°C.

Zur Durchführung der erfindungsgemäßen Verfahrens (a) bzw. (e) setzt man pro Mol an 5-Halogen-1-aryl-pyrazol der Formel (II) im allgemeinen 1,0 bis 1,5 Mol, vorzugsweise 1,0 bis 1,3 Mol an Lithiumorganischer Verbindung und 1,0 bis 10,0 Mol, vorzugsweise 1,0 bis 5,0 Mol an Kohlendioxid oder Dicyan ein.

Dabei setzt man in allgemein üblicher Art und Weise zunächst das 5-Halogen-1-aryl-pyrazol der Formel (II) unter Luft-und Feuchtigkeitsausschluß mit der Lithiumorganischen Verbindung um und leitet anschließend gasförmiges Kohlendioxid in die Reaktionsmischung ein oder gibt in einem geeigneten Lösungsmittel gelöstes Dicyan zu. Die Aufarbeitung und Isolierung der Reaktionsprodukte der Formel (Ia) erfolgt im Fall des erfindungsgemäßen Verfahrens (a) nach allgemein üblichen Verfahren.

Im Fall des erfindungsgemäßen Verfahrens (e) erhält man in der Regel Gemische aus Verbindungen der Formel (Ie1)

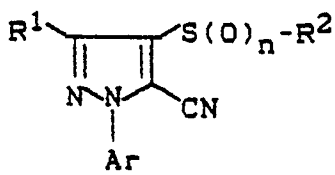

(Ie1)

und Verbindungen der Formel (Ie2)

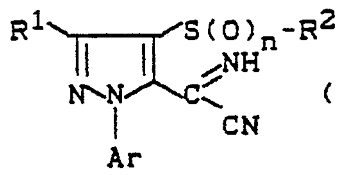

(Ie2)

wobei
$R^1$,$R^2$,Ar und n die oben angegebene Bedeutung haben.

Diese lassen sich mit üblichen Trennmethoden (z.B. säulenchromatographisch) auftrennen.

Als Verdünnungsmittel zur Durchführung des erfindungsgemäßen Verfahrens (b) kommen inerte organische Lösungsmittel in Frage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether wie Diethylether, Dioxan, Tetrahydrofuran oder Ethylenglykoldimethyl-oder -diethylether, Ketone wie Aceton oder Butanon, Nitrile wie Acetonitril oder Propionitril, Amide, wie Dimethylformamid, Dimethylacetamid, N-Methylformanilid, N-Methylpyrrolidon oder Hexamethylphosphorsäuretriamid, Ester wie Essigsäureethylester, oder Sulfoxide, wie Dimethylsulfoxid.
Verwendet man als Reaktionspartner der Formel (III) Alkohole, Amine oder Thiole in flüssiger Form, so ist es auch möglich, diese in einem entsprechenden Überschuß gleichzeitig als Verdünnungsmittel einzusetzen.

Das erfindungsgemäße Verfahren (b) wird gegebenenfalls in Gegenwart eines geeigneten Reaktionshilfsmittels durchgeführt. Als solche kommen prinzipiell alle üblichen für Veresterung und Amidierungen verwendbaren Reaktionshilfsmittel in Frage. Beispielhaft genannt seien Säurehalogenidbildner wie Thionylchlorid, Phosphortrichlorid, Phosphorpentachlorid, Phosphoroxychlorid, oder Aktivesterkomponenten wie N-Hydroxy-Succinimid, Anhydridbildner wie Chlorameisensäure-4-nitrophenylester oder übliche Kondensationsmittel wie Dicyclohexylcarbodiimid (DCC), Triphenylphosphin im Gemisch mit Tetrachlorkohlenstoff, N,N'-Carbonyldiimidazol oder N-Ethoxycarbonyl-2-ethoxy-dihydrochinolin (EEDQ).

Das erfindungsgemäße Verfahren (b) kann gegebenenfalls in Gegenwart eines geeigneten Säurebindemittels durchgeführt werden.
Als solche kommen alle üblichen anorganischen oder organischen Basen in Frage. Hierzu gehören beispielsweise Alkalimetallhydroxide wie Natriumhydroxdid oder Kaliumhydroxid, Alkalimetallcarbonate wie Natriumcarbonat, Kaliumcarbonat oder Natriumhydrogencarbonat, sowie tertiäre Amine wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).
Auch ein entsprechender Überschuß eines gleichzeitig als Reaktionspartner der Formel (III) verwendeten Amins kann gegebenenfalls als Säurebindemittel dienen.

Die Reaktionstemperaturen können bei der Durchführung des erfindungsgemäßen Verfahrens (b) in einem größeren Bereich variiert werden. Im allgemeinen arbeitet man bei Temperaturen zwischen -20°C und +150°C, vorzugsweise bei Temperaturen zwischen 0°C und +100°C.

Zur Durchführung des erfindungsgemäßen Verfahrens (b) setzt man pro Mol an substituierten 1-Arylpyrazol der Formel (Ia) im allgemeinen 1,0 bis 20,0 Mol, vorzugsweise 1,0 bis 10,0 Mol an Alkohol, Amin oder Thiol der Formel (III), 1,0 bis 5,0 Mol, vorzugsweise 1,0 bis 2,0 Mol an Reaktionshilfsmittel und gegebenenfalls 1,0 bis 2,0 Mol an Säurebindemittel ein.
In den meisten Fällen ist es vorteilhaft zunächst aus dem substituierten 1-Arylpyrazol der Formel (Ia) und dem Reaktionshilfsmittel nach üblichen Verfahren einen aktivierten Komplex (Säurehalogenid, Aktivester, gemischtes Säureanhydrid etc.) herzustellen, welcher gegebenenfalls isoliert werden kann und entweder in einem getrennten Reaktionsschritt oder im Eintopfverfahren mit dem Alkohol, Amin oder Thiol der Formel (III) umgesetzt wird. Die Zugabe des Säurebindemittels kann dabei je nach dem verwendeten Reaktionshilfsmittel entweder in der 1.Stufe zur Bildung des aktivierten Komplexes oder in der 2.Stufe zur Umsetzung desselben nützlich sein. Die Reaktionsdurchführung, Aufarbeitung und Isolierung der Reaktionsprodukte der Formel (Ib) erfolgt nach allgemein üblichen Verfahren.

Als Verdünnungsmittel zur Durchführung des erfindungsgemäßen Verfahrens (c) kommen organische oder wässrige Lösungsmittel oder organisch-wässrige Lösungsmittelgemische in Frage. Vorzugsweise verwendet man Alkohole wie Methanol, Ethanol oder Propanol oder deren Gemische mit Wasser sowie reines Wasser als Verdünnungsmittel.

Das erfindungsgemäße Verfahren (c) wird üblicherweise in Gegenwart einer anorganischen oder organischen Base durchgeführt. Als solche verwendet man Hydroxide, Oxide oder Carbonate von Alkali-oder

Erdalkalimetallen oder geeignet substituierte Amine, in Abhängigkeit von der Art des gewünschten Gegenions $M^+$ in den Verbindungen der Formel (Ic).

Die Reaktionstemperaturen können bei der Durchführung des erfindungsgemäßen Verfahrens (c) in einem größeren Bereich variiert werden. Im allgemeinen arbeitet man bei Temperaturen zwischen -20°C und + 120°C, vorzugsweise bei Temperaturen zwischen 0°C und 80°C.

Zur Durchführung des erfindungsgemäßen Verfahrens (c) setzt man pro Mol an substituiertem 1-Arylpyrazol der Formel (Ia1) im allgemeinen 1,0 bis 20,0 Mol, vorzugsweise 1,0 bis 10,0 Mol an Base ein. Die Calcium-, Barium-, Magnesium-, Mangan-, Kupfer-, Nickel-, Zinn-, Eisen-und Cobaltsalze erhält man auch aus den Natriumsalzen durch Behandeln mit einem entsprechenden anorganischen Metallsalz, z.B. Calciumchlorid, Bariumchlorid, Kupfersulfat, Nickelchlorid oder Cobaltnitrat.

Die Aufarbeitung und Isolierung der Salze der Formel (Ic) erfolgt nach üblichen Methoden.

Als Verdünnungsmittel zur Durchführung des erfindungsgemäßen Verfahrens (d) kommen inerte organische Lösungsmittel in Frage. Hierzu gehören insbesondere aliphatische oder aromatische, gegebenenfalls halogenierte Kohlenwasserstoffe wie beispielsweise Benzin, Benzol, Toluol, Xylol, Chlorbenzol, Petrolether, Hexan, Cyclohexan, Dichlormethan, Chloroform, Tetrachlorkohlenstoff, Ether wie Diethylether, Dioxan, Tetrahydrofuran oder Ethylenglykoldimethyl-oder -diethylether, Ketone wie Aceton oder Butanon, Nitrile wie Acetonitril oder Propionitril, Amide wie Dimethylformamid, Dimethylacetamid, N-Methylformanilid, N-Methylpyrrolidon oder Hexamethylphosphorsäuretriamid, Ester wie Essigsäureethylester oder Sulfoxide wie Dimethylsulfoxid.

Die Reaktionstemperaturen können bei der Durchführung des erfindungsgemäßen Verfahrens (d) in einem größeren Bereich variiert werden. Im allgemeinen arbeitet man bei Temperaturen zwischen 0°C und +180°C, vorzugsweise bei Temperaturen zwischen +20°C und +150°C.

Zur Durchführung des erfindungsgemäßen Verfahrens (d) setzt man pro Mol an substituiertem 1-Arylpyrazol der Formel (Ic) im allgemeinen 1,0 bis 10,0 Mol, vorzugsweise 1,0 bis 5,0 Mol an Alkylierungsmittel der Formel (IV) ein. Die Reaktionsdurchführung, Aufarbeitung und Isolierung der Reaktionsprodukte der Formel (Id) erfolgt nach üblichen und bekannten Verfahren.

Als Oxidationsmittel zur Durchführung des erfindungsgemäßen Verfahrens (f) kommen alle üblichen zur Schwefeloxidation verwendbaren Oxidationsmittel infrage. Insbesondere geeignet sind Wasserstoffperoxid, organische Persäuren, wie beispielsweise Peressigsäure, m-Chlorperbenzoesäure, p-Nitroperbenzoesäure oder Luftsauerstoff.

Als Verdünnungsmittel zur Durchführung des erfindungsgemäßen Verfahrens (f) kommen ebenfalls inerte organische Lösungsmittel in Frage. Vorzugsweise verwendet man Kohlenwasserstoffe, wie Benzin, Benzol, Toluol, Hexan oder Petrolether; chlorierte Kohlenwasserstoffe, wie Dichlormethan, 1,2-Dichlorethan, Chloroform, Tetrachlorkohlenstoff oder Chlorbenzol; Ether, wie Diethylether, Dioxan oder Tetrahydrofuran; Carbonsäuren, wie Essigsäure oder Propionsäure, oder dipolar aprotische Lösungsmittel, wie Acetonitril, Aceton, Essigsäureethylester oder Dimethylformamid.

Das erfindungsgemäße Verfahren (f) kann gegebenenfalls in Gegenwart eines Säurebindemittels durchgeführt werden. Als solche kommen alle üblicherweise verwendbaren organischen und anorganischen Säurebindemittel in Frage. Vorzugsweise verwendet man Erdalkali-oder Alkalimetallhydroxide, -acetate oder -carbonate, wie beispielsweise Calciumhydroxid, Natriumhydroxid, Natriumacetat oder Natriumcarbonat.

Das erfindungsgemäße Verfahren (f) kann gegebenenfalls in Gegenwart eines geeigneten Katalysators durchgeführt werden. Als solche kommen alle üblicherweise für derartige Schwefeloxidationen gebräuchlichen Metallsalz-Katalysatoren in Frage. Beispielhaft genannt sei in diesem Zusammenhang Ammoniummolybdat.

Die Reaktionstemperaturen können bei der Durchführung des erfindungsgemäßen Verfahrens (f) in einem größeren Bereich variiert werden. Im allgemeinen arbeitet man bei Temperaturen zwischen -20 °C und +70 °C, vorzugsweise bei Temperaturen zwischen 0 °C und +50 °C.

Zur Durchführung des erfindungsgemäßen Verfahrens (f) setzt man pro Mol an substituiertem 1-Arylpyrazol der Formel (Ig) im allgemeinen 0.8 bis 1.2 Mol, vorzugsweise äquimolare Mengen Oxidationsmittel ein, wenn man die Oxidation des Schwefels auf der Sulfoxidstufe unterbrechen will. Zur Oxidation zum Sulfon setzt man pro Mol an substituierten 1-Arylpyrazol der Formel (Ig) im allgemeinen 1.8 bis 3.0 Mol, vorzugsweise doppelt molare Mengen an Oxidationsmittel ein. Die Reaktionsführung, Aufarbeitung und Isolierung der Endprodukte der Formel (If) erfolgt nach üblichen Verfahren.

Die Wirkstoffe eignen sich zur Bekämpfung von tierischen Schädlingen, vorzugsweise Arthropoden, insbesondere Insekten, die in der Landwirtschaft, in Forsten, im Vorrats-und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp.. Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Die erfindungsgemäßen Wirkstoffe zeichnen sich durch eine hohe insektizide Wirksamkeit aus. Sie lassen sich mit besonders gutem Erfolg gegen pflanzenschädigende Insekten, wie beispielsweise gegen die Larven der Meerettichblattkäfer (Phaedon cochleariae) einsetzen. Dabei zeigen die erfindunsggemäßen Wirkstoffe neben guten protektiven auch hervorragende systemische Eigenschaften. Daneben eignen sie sich auch besonders gut zur Bekämpfung von Bodeninsekten und lassen sich beispielsweise zur Bekämpfung von Phorbia antiqua Maden im Boden einsetzen. Darüberhinaus eignen sie sich auch zur Bekämpfung von Hygiene-und Vorratsschädlingen.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur-und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergier mitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder - schaumerzeugende Mittel kommen in Frage: z.B. nichtionogene und anionische Emul gatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Die Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Herstellungsbeispiele:

Beispiel 1:

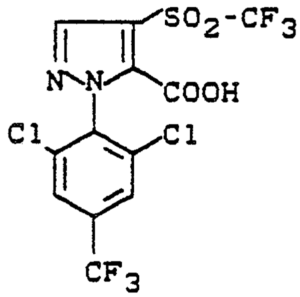

(Verfahren a)

Zu 29,5 g (0,06 Mol) 5-Brom-4-trifluormethyl-sulfonyl-1-(2,6-dichlor-4-trifluormethylphenyl)-pyrazol (vergl. DE-OS 35 29 829) in 200 ml absolutem Ether gibt man bei -78°C tropfenweise unter einer Stickstoffatmosphäre 42 ml (0,066 Mol) einer 15%igen n-Butyllithiumlösung in n-Hexan, rührt anschließend 2 Stunden bei -78°C und leitet dann einen Überschuß an gasförmigen Kohlendioxid ein, wobei man die Reaktionsmischung langsam auf Raumtemperatur erwärmen läßt.

Zur Aufarbeitung versetzt man mit 300 ml Wasser, säuert unter Eiskühlung vorsichtig an, extrahiert dreimal mit Dichlormethan, trocknet über Magnesiumsulfat und entfernt das Lösungsmittel im Vakuum.

Man erhält 19,6 g (72 % der Thoerie) an 1-(2,6-Dichlor-4-trifluormethylphenyl)-4-trifluormethylsulfonyl-pyrazol-5-yl-carbonsäure vom Schmelzpunkt 158°C (Zers.).

Beispiel 2:

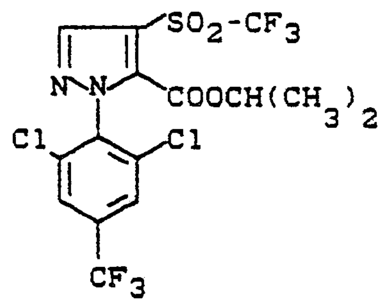

(Verfahren b)

3,42 g (0,0075 Mol) 1-(2,6-Dichlor-4-trifluormethylphenyl)-4-trifluormethylsulfonyl-pyrazol-5-yl-carbonsäure und 1,56 g (0,0075 Mol) Phosphorpentachlorid werden in 30 ml absolutem Ether 15 Minuten auf Rückflußtemperatur erwärmt, im Vakuum eingeengt und mit 30ml Isopropanol sowie 0,75 g (0,005 Mol) Triethylamin versetzt. Die Mischung wird 16 Stunden bei Raumtemperatur gerührt, im Vakuum eingeengt, der Rückstand in Dichlormethan aufgenommen, mit Wasser gewaschen, über Magnesiumsulfat getrocknet, im Vakuum eingeengt und chromatographisch (Kieselgel; Laufmittel Dichlormethan/Hexan = 7:3) gereinigt.

Man erhält 2,4 g (65 % der Theorie) an 1-(2,6-Dichlor-4-trifluormethylphenyl)-4-trifluormethylsulfonyl-pyrazol-5-yl-carbonsäureisopropylester vom Schmelzpunkt 81-82°C.

Beispiel 3:

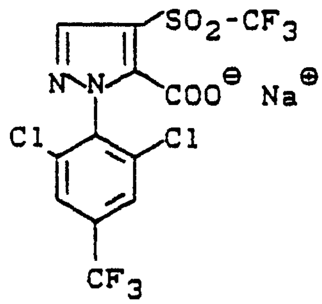

(Verfahren c)

2,28 g (0,005 Mol) 1-(2,6-Dichlor-4-trifluormethylphenyl)-4-trifluormethylsulfonyl-pyrazol-5-yl-carbonsäure und 0,2 g (0,005 Mol) Natriumhydroxid in 10 ml Wasser werden 1 Stunde bei Raumtemperatur gerührt und im Vakuum eingeengt. Man erhält 2,3 g (96 % der Theorie) an 1-(2,6-Dichlor-4-trifluormethylphenyl)-4-trifluormethylsulfonylpyrazol-5-yl-carbonsäure Natriumsalz vom Schmelzpunkt >200°C.

Beispiel 4 / 5:

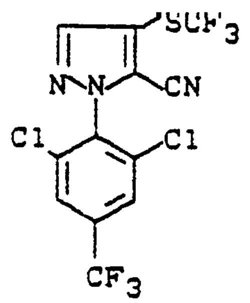

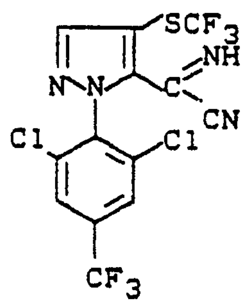

(Verfahren e)

19,3 g (0,042 Mol) 5-Brom-4-trifluormethylsulfenyl-1-(2,6-dichlor-4-trifluormethylphenyl)-pyrazol (vergl. DE-OS 35 29 829) in 150 ml absolutem Ether werden unter einer Argonatmosphäre bei -78°C tropfenweise unter Rühren mit 28 ml (0,046 Mol) einer 15%-igen n-Butyllithiumlösung in n-Hexan versetzt, 2 Stunden bei -78°C gerührt, dann mit 3,2 g (0,061 Mol) Dicyan in 50 ml Ether versetzt und langsam auf Raumtemperatur erwärmt. Zur Aufarbeitung hydrolysiert man mit Wasser, säuert an, extrahiert 2 mal mit Dichlormethan, trocknet über Magnesiumsulfat, engt im Vakuum ein und trennt das Rohproduktgemisch säulenchromatographisch (Kieselgel; Dichlormethan/Hexan = 7:3).

Man erhält 6 g (35,2 % der Theorie) an 1-(2,6-Dichlor-4-trifluormethylphenyl)-4-trifluormethylsulfenyl-pyrazol-5-yl-carbonitril vom Schmelzpunkt 40-45°C und 5 g (27,4 % der Theorie) an α-Imino-[1-(2,6-Dichlor-4-trifluormethylphenyl)-4-trifluormethylsulfenyl-pyrazol-5-yl]-acetonitril vom Schmelzpunkt 128-130°C.

In entsprechender Weise und gemäß den allgemeinen Angaben zur Herstellung erhält man die folgenden substituierten 1-Arylpyrazole der allgemeinen Formel (I):

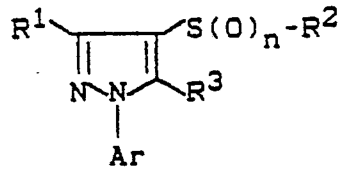

(I)

| Bsp. Nr. | $R^1$ | $-S(O)_n-R^2$ | $R^3$ | Ar | physikal. Eigen-schaften |
|---|---|---|---|---|---|
| 6 | $CH_3$ | $-SCCl_2F$ | $COOCH_3$ | 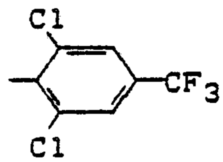 | Fp:205-206° C |
| 7 | $CH_3$ | $-SCF_3$ | COOH | 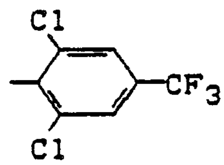 | Fp:214-215° C |
| 8 | H | $-SO_2CCl_2F$ | COOH | 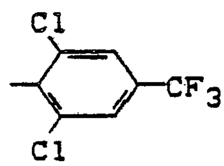 | Fp:174-176° C |
| 9 | H | $-SO_2-CF_3$ | $COOCH_3$ | 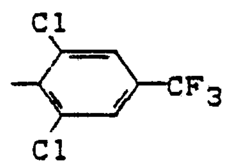 | Fp:58-59° C |

| Bsp. Nr. | $R^1$ | $-S(O)_n-R^2$ | $R^3$ | Ar | physikal. Eigenschaften |
|---|---|---|---|---|---|
| 10 | H | $-SO_2-CF_3$ | $COOC_2H_5$ | 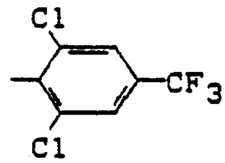 | $n_D^{20}$ : 1,491 |
| 11 | H | $-SCH_3$ | COOH | 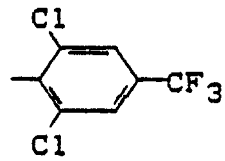 | Fp: 167-168° C |
| 12 | H | $-SO_2CCl_2F$ | $COOC_2H_5$ | 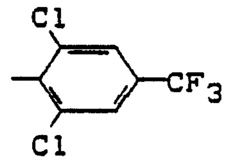 | $n_D^{20}$ : 1,527 |
| 13 | $CH_3$ | $-SO_2-CF_3$ | COOH | 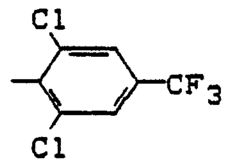 | Fp: 183-184° C |
| 14 | H | $-S-CF_3$ | $COOCH_3$ | 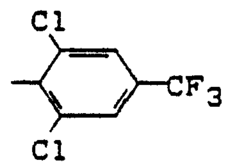 | Fp: 87-88° C |
| 15 | H | $-S-CF_3$ | $COOC_2H_5$ | 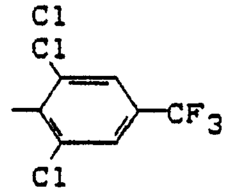 | $n_D^{20}$ : 1,4803 |
| 16 | H | $-S-CF_3$ | $COOCH(CH_3)_2$ | 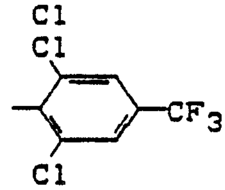 | $n_D^{20}$ : 1,4814 |
| 17 | H | $-SCF_3$ | $COO^{\ominus}Na^{\oplus}$ | 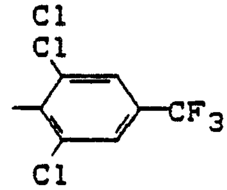 | Fp: 125-126° C |
| 18 | H | $-SCF_3$ | $CO-NHC_2H_5$ | 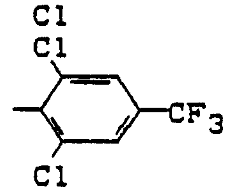 | Fp: 133-134° C |
| 19 | $CH_3$ | $-SCCl_2F$ | $COOCH_3$ | 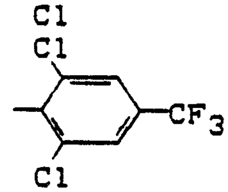 | Fp: 120-121° C |

Anwendungsbeispiele:

In den folgenden Anwendungsbeispielen wurden die nachstehend aufgeführten Verbindungen als Vergleichssubstanzen eingesetzt:

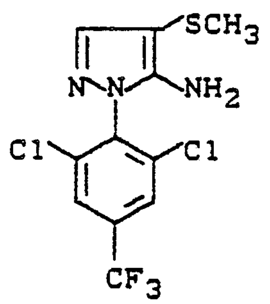

(A)

**5-Amino-1-(2,6-dichlor-4-trifluormethylphenyl)-5-methylthio-pyrazol**

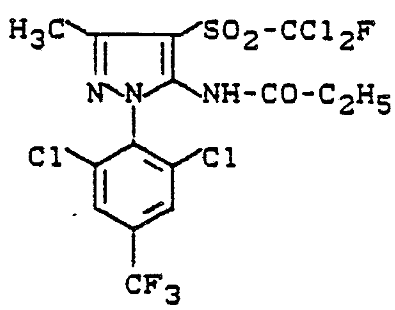

(B)

**3-Methyl-4-fluordichlormethylsulfonyl-1-(2,6-dichlor-4-trifluormethylphenyl)-5-propionamido-pyrazol**

**(beide bekannt aus EP-A 201 852)**

Beispiel A

Phaedon-Larven-Test

Lösungsmittel: 7 Gewichtsteile Dimethylformamid

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käfer-Larven abgetötet wurden.

Bei diesem Test zeigen z.B. die folgenden Verbindungen der Herstellungsbeispiele überlegene Wirksamkeit gegenüber dem Stand der Technik: 8, 9, 10 und 12.

Beispiel B

Grenzkonzentrations-Test / Bodeninsekten
Testinsekt: Phorbia antiqua-Maden (im Boden)
Lösungsmittel: 3 Geweichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffes in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigen z.B. die folgenden Verbindungen der Herstellungsbeispiele überlegene Wirksamkeit gegenüber dem Stand der Technik: 1, 2, 6, 8, 9, 12 und 18.

Beispiel C

Grenzkonzentrations-Test / Wurzelsystemische Wirkung
Testinsekt: Phaedon cochleariae
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinhet Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 1, 2, 3 und 8.

**Ansprüche**

1. Substituierte 1-Arylpyrazole der allgemeinen Formel (I),

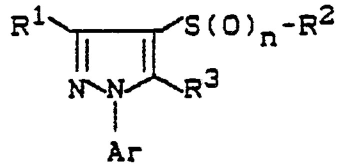

(I)

in welcher
$R^1$ für Wasserstoff, Alkyl oder Halogenalkyl steht,
$R^2$ für Alkyl, Alkenyl, Alkinyl, Cycloalkyl, Halogenalkyl, Halogenalkenyl, Alkoxyalkyl, Alkylthioalkyl, Alkylsulfinylalkyl, Alkylsulfonylalkyl, für gegebenenfalls substituiertes Aralkyl oder für gegebenenfalls substituiertes Aryl steht,
n für die Zahlen 0, 1 oder 2 steht,
$R^3$ für Cyano, für einen Rest

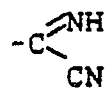

oder für einen Rest $-\underset{O}{\underset{\|}{C}}-Y-R^4$ steht und
Ar für substituiertes Phenyl oder für gegebenenfalls substituiertes Pyridyl steht, wobei
Y für Sauerstoff, Schwefel oder einen Rest $-\underset{|}{N}-R^5$ steht
$R^4$ für Wasserstoff, für Alkyl, Hydroxyalkyl, Alkoxyalkyl, Alkylthioalkyl, Halogenalkyl, Alkenyl, Halogenalkenyl, Alkinyl oder für jeweils gegebenenfalls substituiertes Cycloalkyl, Aralkyl oder Aryl steht, für einen Rest $-A-\underset{O}{\underset{\|}{C}}-Z-R^6$ steht, oder für den Fall, daß Y für Sauerstoff, Schwefel oder einen Rest $\underset{SO_2-R^7}{\underset{|}{-N-}}$ steht, auch für ein salzartig gebundenes Kation steht,
$R^5$ für Wasserstoff, für Alkyl, Hydroxyalkyl, Alkoxyalkyl, Alkylthioalkyl, Halogenalkyl, Alkenyl, Alkinyl oder Halogenalkenyl, für jeweils gegebenenfalls substituiertes Cycloalkyl, Aralkyl oder Aryl steht oder für den Rest $-SO_2-R^7$ steht,
A für einen zweifach verknüpften Alkenylrest steht,
Z für Sauerstoff, Schwefel oder einen N-Alkylrest steht,
$R^6$ für Wasserstoff, Alkyl, Alkenyl oder Alkinyl steht und
$R^7$ für jeweils gegebenenfalls substituiertes Alkyl, Aralkyl oder Aryl steht.

2. Substituierte 1-Arylpyrazole gemäß Anspruch 1, Formel (I) in welcher
$R^1$ für Wasserstoff, oder für jeweils geradkettiges oder verzweigtes Alkyl oder Halogenalkyl mit 1 bis 4 Kohlenstoffatomen und gegebenenfalls 1 bis 9 gleichen oder verschiedenen Halogenatomen steht,
$R^2$ für jeweils geradkettiges oder verzweigtes Alkyl, Alkenyl oder Alkinyl mit jeweils bis zu 8 Kohlenstoffatomen, für Cycloalkyl mit 3 bis 7 Kohlenstoffatomen, für jeweils geradkettiges oder verzweigtes Halogenalkyl oder Halogenalkenyl mit jeweils bis zu 8 Kohlenstoffatomen und bis zu 17 gleichen oder verschiedenen Halogenatomen, für jeweils geradkettiges oder verzweigtes Alkoxyalkyl, Alkylthioalkyl, Alkylsulfinylalkyl oder Alkylsulfonylalkyl mit jeweils 1 bis 6 Kohlenstoffatomen in den einzelnen Alkylteilen oder für jeweils im Phenylteil gegebenenfalls einfach oder mehrfach, gleich oder verschieden substituiertes Phenylalkyl oder Phenyl mit gegebenenfalls 1 bis 4 Kohlenstoffatomen im geradkettigen oder verzweigten Alkylteil steht, wobei als Substituenten im Phenylteil infrage kommen: Halogen, Cyano, Nitro, jeweils geradkettiges oder verzweigtes Alkyl, Alkoxy, Alkylthio, Alkylsulfinyl, Alkylsulfonyl, Halogenalkyl, Halogenalkoxy, Halogenalkylthio, Halogenalkylsulfinyl oder Halogenalkylsulfonyl mit jeweils 1 bis 4 Kohlenstoffatomen in den einzelnen Alkylteilen und gegebenenfalls 1 bis 9 gleichen oder verschiedenen Halogenatomen,
n für eine Zahl 0, 1 oder 2 steht,
$R^3$ für Cyano, für einen Rest

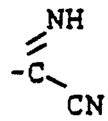

oder für einen Rest $-\underset{\underset{O}{\|}}{C}-X-R^4$ steht und

Ar für einfach oder mehrfach, gleich oder verschieden substituiertes Phenyl oder für jeweils gegebenenfalls einfach oder mehrfach gleich oder verschieden substituiertes 2-Pyridyl, 3-Pyridyl oder 4-Pyridyl steht, wobei als Substituenten jeweils in Frage kommen: Cyano, Nitro, Halogen, jeweils geradkettiges oder verzweigtes Alkyl, Alkoxy oder Alkoxycarbonyl mit jeweils 1 bis 4 Kohlenstoffatomen, außerdem jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $-S(O)_p-R^8$,

wobei

Y für Sauerstoff, Schwefel oder für einen Rest $-N-R^5$ steht,

$R^4$ für Wasserstoff, für jeweils geradkettiges oder verzweigtes Alkyl, Hydroxyalkyl, Alkoxyalkyl, Alkylthioalkyl, Halogenalkyl, Alkenyl, Halogenalkenyl oder Alkinyl mit jeweils bis zu 12 Kohlenstoffatomen und im Fall des Halogenalkyl bzw. des Halogenalkenyl mit 1 bis 15 gleichen oder verschiedenen Halogenatomen, für Cycloalkyl mit 3 bis 7 Kohlenstoffatomen oder für jeweils gegebenenfalls einfach oder mehrfach, gleich oder verschieden durch Halogen, Cyano, Nitro sowie jeweils geradkettiges oder verzweigtes Alkyl, Alkoxy, Alkylthio oder Halogenalkyl mit jeweils 1 bis 4 Kohlenstoffatomen und im Fall des Halogenalkyls mit 1 bis 9 gleichen oder verschiedenen Halogenatomen substituiertes Phenyl, Benzyl oder Phenethyl steht, außerdem für einen Rest $-A-\underset{\underset{O}{\|}}{C}-Z-R^6$ steht oder

für den Fall, daß Y für Sauerstoff, Schwefel oder einen Rest $-\underset{\underset{SO_2-R^7}{|}}{N}-$ steht,

auch für ein Äquivalent eines Alkali-, Erdalkali-, Kupfer-, Zink-, Mangan-, Zinn-, Eisen-, Cobalt-oder Nickelkations oder für ein gegebenenfalls einfach oder mehrfach, gleich oder verschieden substituiertes Ammonium-, Phosphonium-oder Sulfoniumkation steht, wobei als Substituenten in Frage kommen: geradkettiges oder verzweigtes Alkyl mit 1 bis 18 Kohlenstoffatomen, Phenyl oder Benzyl,

$R^5$ für Wasserstoff, für jeweils geradkettiges oder verzweigtes Alkyl, Hydroxyalkyl, Alkoxyalkyl, Alkylthioalkyl, Halogenalkyl, Alkenyl, Halogenalkenyl oder Alkinyl mit jeweils bis zu 12 Kohlenstoffatomen und im Falle des Halogenalkyl bzw. des Halogenalkenyl mit 1 bis 15 gleichen oder verschiedenen Halogenatomen, für Cycloalkyl mit 3 bis 7 Kohlenstoffatomen oder für jeweils gegebenenfalls einfach oder mehrfach, gleich oder verschieden durch Halogen, Cyano, Nitro sowie jeweils geradkettiges oder verzweigtes Alkyl, Alkoxy, Alkylthio oder Halogenalkyl mit jeweils 1 bis 4 Kohlenstoffatomen und im Fall des Halogenalkyls mit 1 bis 9 gleichen oder verschiedenen Halogenatomen substituiertes Phenyl, Benzyl oder Phenethyl steht, außerdem für einen Rest $-SO_2-R^7$ steht,

A für einen zweifach verknüpften geradkettigen oder verzweigten Alkylrest mit 1 bis 8 Kohlenstoffatomen steht,

Z für Sauerstoff, Schwefel oder einen N-Alkylrest mit 1 bis 4 Kohlenstoffatomen im geradkettigen oder verzweigten Alkylteil steht,

$R^6$ für Wasserstoff oder für jeweils geradkettiges oder verzweigtes Alkyl, Alkenyl oder Alkinyl mit jeweils bis zu 6 Kohlenstoffatomen steht,

$R^7$ für jeweils geradkettiges oder verzweigtes Alkyl oder Halogenalkyl mit jeweils 1 bis 6 Kohlenstoffatomen und im Fall des Halogenalkyls mit 1 bis 9 gleichen oder verschiedenen Halogenatomen oder für gegebenenfalls einfach oder mehrfach, gleich oder verschieden substituiertes Phenyl, Benzyl oder Phenylethyl steht, wobei als Substituenten jeweils in Frage kommen: Halogen, Cyano, Nitro, jeweils geradkettiges oder verzweigtes Alkyl, Alkoxy, Alkylthio oder Halogenalkyl mit jeweils 1 bis 4 Kohlenstoffatomen und im Fall des Halogenalkyl mit 1 bis 9 gleichen oder verschiedenen Halogenatomen,

$R^8$ für Amino sowie für jeweils geradkettiges oder verzweigtes Alkyl, Alkylamino, Dialkylamino oder Halogenalkyl mit jeweils 1 bis 4 Kohlenstoffatomen in den einzelnen Alkylteilen und im Fall des Halogenalkyl mit 1 bis 9 gleichen oder verschiedenen Halogenatomen steht und

p für eine Zahl 0, 1 oder 2 steht.

3. Substituierte 1-Arylpyrazole gemäß Anspruch 1, Formel (I), in welcher
$R^1$ für Wasserstoff, Methyl, Ethyl, n-oder i-Propyl, n-, i-, s-oder t-Butyl oder Trifluormethyl steht,
$R^2$ für Methyl, Ethyl, n-oder i-Propyl, n-, i-, s-oder t-Butyl, n-oder i-Pentyl, n-oder i-Hexyl, Allyl, n-oder i-Butenyl, n-oder i-Pentenyl, Propargyl, n-oder i-Butinyl, Cyclopropyl, Cyclobutyl, Cyclopentyl, Cyclohexyl, Chlormethyl, Difluormethyl, Difluorchlormethyl, Fluordichlormethyl, Trifluormethyl, Pentafluorethyl, Pentachlorethyl, Fluortetrachlorethyl, Difluortrichlorethyl, Trifluordichlorethyl, Tetrafluorchlorethyl, Heptafluorpropyl, Chlorethyl, Bromethyl, Chlorpropyl, Brompropyl, Dichlormethyl, Chlorfluormethyl, Trichlormethyl, Trifluorethyl, Trifluorchlorethyl, Tetrafluorethyl, Difluorchlorethyl, Fluordibrommethyl, Difluorbrommethyl, Fluorchlorbrommethyl, Chlorallyl, Fluorallyl, Chlorbutenyl, Fluorbutenyl, Dichlorallyl, Fluorchlorallyl, Difluorallyl, Bromallyl, für Methoxymethyl, Methoxyethyl, Methoxypropyl, Ethoxymethyl, Ethoxyethyl, Methylthiomethyl, Methylsulfinylmethyl, Methylsulfonylethyl, Methylthioethyl, Methylthiopropyl, Methylsulfinylethyl, Methylsulfonylmethyl oder für jeweils gegebenenfalls einfach bis dreifach, gleich oder verschieden substituiertes Phenyl, Benzyl oder Phenylethyl steht, wobei als Phenylsubstituenten jeweils in Frage kommen: Fluor, Chlor, Brom, Iod, Cyano, Nitro, Methyl, Ethyl, Methoxy, Methylthio, Trifluormethyl, Methylsulfinyl, Methylsulfonyl, Trifluormethoxy, Trifluormethylthio, Trifluormethylsulfinyl oder Trifluormethylsulfonyl,
n für eine Zahl 0, 1 oder 2 steht,
$R^3$ für Cyano, für einen Rest

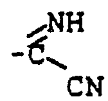

oder für einen Rest $-\underset{\underset{O}{\|}}{C}-Y-R^4$ steht und
Ar für ein-bis fünffach, gleich oder verschieden substituiertes Phenyl oder für jeweils gegebenenfalls ein-bis vierfach gleich oder verschieden substituiertes 2-Pyridyl oder 4-Pyridyl steht, wobei als Phenyl-bzw. Pyridylsubstituenten jeweils in Frage kommen: Cyano, Nitro, Fluor, Chlor, Brom, Iod, Methyl, Ethyl, n-und i-Propyl, n-, i-, s-und t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Tri chlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder einen Rest $-S(O)_p-R^8$,
wobei
Y für Sauerstoff, Schwefel oder einen Rest $-\underset{|}{N}-R^5$ steht,
$R^4$ für Wasserstoff, für gegebenenfalls ein-bis dreifach, gleich oder verschieden durch Fluor, Chlor, Hydroxy, Methoxy, Ethoxy, Methylthio oder Ethylthio substituiertes Methyl, Ethyl, n-oder i-Propyl, n-, i-, s-oder t-Butyl, n-oder i-Pentyl, für gegebenenfalls ein-bis dreifach, gleich oder verschieden durch Fluor oder Chlor substituiertes Allyl, Propenyl oder Butenyl, für Propargyl, Propinyl, Butinyl oder Pentinyl, für Cyclopropyl, Cyclopentyl, Cyclohexyl, für jeweils gegebenenfalls ein-bis fünffach, gleich oder verschieden durch Fluor, Chlor, Brom, Cyano, Nitro, Methyl, Ethyl, Methoxy, Ethoxy, Methylthio oder Trifluormethyl substituiertes Benzyl oder Phenyl steht, für einen Rest $-A-\underset{\underset{O}{\|}}{C}-Z-R^6$ steht,
und außerdem für den Fall, daß Y für Sauerstoff, Schwefel oder einen Rest $-\underset{\underset{SO_2-R^7}{|}}{N}-$ steht,
auch für ein Äquivalent eines Natrium-, Kalium-, Magnesium-, Calcium-, Barium-, Kupfer-, Zink-, Mangan-, Zinn-, Eisen-, Cobalt-oder Nickelkations oder für ein gegebenenfalls ein-bis dreifach, gleich oder verschieden durch Methyl, Ethyl, n-oder i-Propyl, n-, i-, s-oder t-Butyl, Benzyl oder Phenyl substituiertes Ammonium-, Phosphonium-oder Sulfoniumkation steht,
$R^5$ für Wasserstoff, für gegebenenfalls ein-bis dreifach, gleich oder verschieden durch Fluor, Chlor, Hydroxy, Methoxy, Ethoxy, Methylthio oder Ethylthio substituiertes Methyl, Ethyl, n-oder i-Propyl, n-, i-, s-oder t-Butyl, n-oder i-Pentyl, für gegebenenfalls ein-bis dreifach, gleich oder verschieden durch Fluor oder Chlor substituiertes Allyl, Propenyl oder Butenyl, für Propargyl, Propinyl, Butinyl oder Pentinyl, für Cyclopropyl, Cyclopentyl, Cyclohexyl, für jeweils gegebenenfalls ein-bis fünffach, gleich oder verschieden durch Fluor, Chlor, Brom, Cyano, Nitro, Methyl, Ethyl, Methoxy, Ethoxy, Methylthio oder Trifluormethyl

substituiertes Benzyl oder Phenyl und außerdem für einen Rest $-SO_2-R^7$ steht,
A für einen zweifach verknüpften geradkettigen oder verzweigten Alkylenrest mit 1 bis 6 Kohlenstoffatomen steht,
Z für Sauerstoff, Schwefel, einen N-Methyl-oder einen N-Ethylrest steht,
$R^6$ für Wasserstoff, Methyl, Ethyl, n-oder i-Propyl, n-, i-, s-oder t-Butyl, für Allyl, Propenyl, Butenyl, Propargyl, Propinyl oder Butinyl steht,
$R^7$ für Methyl, Ethyl, n-oder i-Propyl, n-, i-, s-oder t-Butyl oder für gegebenenfalls ein-bis fünffach, gleich oder verschieden durch Fluor, Chlor, Brom, Cyano, Nitro, Methyl, Ethyl, Methoxy, Methylthio oder Trifluormethyl substituiertes Benzyl oder Phenyl steht,
$R^8$ für Amino, Methylamino, Ethylamino, Dimethylamino, Diethylamino, Fluordichlormethyl, Difluorchlormethyl, Tetrafluorethyl, Trifluorchlorethyl, Trifluormethyl, Methyl oder Ethyl steht und
p für eine Zahl 0, 1 oder 2 steht.

4. Substituierte 1-Arylpyrazole gemäß Anspruch 1, Formel (I), in welcher
$R^1$ für Wasserstoff, Methyl, t-Butyl oder Trifluormethyl steht,
$R^2$ für Methyl, Ethyl, Trifluormethyl, Dichlorfluormethyl oder Difluorchlormethyl steht,
n für eine Zahl 0, 1 oder 2 steht,
$R^3$ für Cyano, für einen Rest

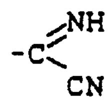

oder für einen Rest $-\underset{\underset{O}{\|}}{C}-Y-R^4$ steht und
Ar für ein-bis fünffach, gleich oder verschieden substituiertes Phenyl oder für jeweils gegebenenfalls ein-bis vierfach gleich oder verschieden substituiertes 2-Pyridyl oder 4-Pyridyl steht, wobei als Phenyl-bzw. Pyridylsubstituenten jeweils in Frage kommen: Cyano, Nitro, Fluor, Chlor, Brom, Iod, Methyl, Ethyl, n-und i-Propyl, n-, i-, s-und t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluor chlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder einen Rest $-S(O)_p-R^8$, wobei
Y für Sauerstoff, Schwefel oder für einen Rest $-\underset{|}{N}-R^5$ steht,
$R^4$ für Methyl, Ethyl, n-oder i-Propyl, n-, i-, s-oder t-Butyl, n-oder i-Pentyl, Allyl, Propenyl, n-oder i-Butenyl, n-oder i-Pentenyl, Propargyl, Propinyl, n-oder i-Butinyl, n-oder i-Pentinyl, Trifluorethyl, Trichlorethyl, Chlorethyl, Chlorpropenyl, Dichlorpropenyl, Hydroxyethyl, Hydroxypropyl, Methoxymethyl, Methoxyethyl, Methoxypropyl, Ethoxymethyl, Ethoxyethyl, Ethoxypropyl, Propoxymethyl, Propoxyethyl, Butoxymethyl, Butoxyethyl, Methylthiomethyl, Methylthioethyl, Methylthiopropyl, Ethylthioethyl, Ethylthiopropyl oder Propylthioethyl steht, und außerdem für den Fall, daß Y für einen Rest $-\underset{\underset{SO_2-R^7}{|}}{N}-$ steht, auch für ein Natrium-oder Kaliumion oder für ein gegebenenfalls ein-bis vierfach, gleich oder verschieden durch Methyl, Ethyl, n-oder i-Propyl, n-Butyl oder Benzyl substituiertes Ammoniumion steht,
$R^5$ für Wasserstoff, Methyl, Ethyl, n-oder i-Propyl, n-, i-, s-oder t-Butyl, n-oder i-Pentyl, Allyl, Propenyl, n-oder i-Butenyl, n-oder i-Pentenyl, Propargyl, Propinyl, n-oder i-Butinyl, n-oder i-Pentinyl, Trifluorethyl, Trichlorethyl, Chlorethyl, Chlorpropenyl, Dichlorpropenyl, Hydroxyethyl, Hydroxypropyl, Methoxymethyl, Methoxyethyl, Methoxypropyl, Ethoxymethyl, Ethoxyethyl, Ethoxypropyl, Methylthiomethyl, Methylthioethyl, Methylthiopropyl, Ethylthioethyl oder Ethylthiopropyl steht, oder für einen Rest $-SO_2-R^7$ steht, wobei
$R^7$ für Methyl, Ethyl, p-Tolyl oder Phenyl steht,
$R^8$ für Amino, Methylamino, Ethylamino, Dimethylamino, Diethylamino, Fluordichlormethyl, Difluorchlormethyl, Tetrafluorethyl, Trifluorchlorethyl, Trifluormethyl, Methyl oder Ethyl steht und
p für ein Zahl 0, 1 oder 2 steht.

5. Substituierte 1-Arylpyrazole gemäß Anspruch 1, Formel (I), in welcher
$R^1$ für Wasserstoff, Methyl, t-Butyl oder Trifluormethyl steht,
$R^2$ für Methyl, Ethyl, Trifluormethyl, Dichlorfluormethyl oder Difluorchlormethyl steht,

n für eine Zahl 0, 1 oder 2 steht,
$R^3$ für einen Rest $-\underset{\underset{O}{\|}}{C}-Y-R^4$ steht und
Ar für ein-bis fünffach, gleich oder verschieden substituiertes Phenyl oder für jeweils gegebenenfalls ein-bis vierfach gleich oder verschieden substituiertes 2-Pyridyl oder 4-Pyridyl steht, wobei als Phenyl-bzw. Pyridylsubstituenten jeweils in Frage kommen: Cyano, Nitro, Fluor, Chlor, Brom, Iod, Methyl, Ethyl, n-und i-Propyl, n-, i-, s-und t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder einen Rest $-S(O)_p-R^8$,
wobei
Y für Sauerstoff oder Schwefel steht,
$R^4$ für Wasserstoff oder für ein Äquivalent eines Natrium-, Kalium-, Calcium-, Magnesium-, Barium-, Kupfer-, Zink-, Mangan-, Zinn-, Eisen-, Cobalt-oder Nickelkations steht oder für ein gegebenenfalls ein-bis vierfach, gleich oder verschieden durch Methyl, Ethyl, n-oder i-Propyl, n-, i-oder s-Butyl, Benzyl oder Phenyl substituiertes Ammoniumkation steht,
$R^8$ für Amino, Methylamino, Ethylamino, Dimethylamino, Diethylamino, Fluordichlormethyl, Difluorchlormethyl, Tetrafluorethyl, Trifluorchlorethyl, Trifluormethyl, Methyl oder Ethyl steht und
p für ein Zahl 0, 1 oder 2 steht.

6. Verfahren zur Hestellung von substituierten 1-Arylpyrazolen der allgemeinen Formel (I)

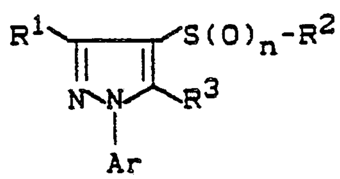

(I)

in welcher
$R^1$ für Wasserstoff, Alkyl oder Halogenalkyl steht, $R^2$ für Alkyl, Alkenyl, Alkinyl, Cycloalkyl, Halogenalkyl, Halogenalkenyl, Alkoxyalkyl, Alkylthioalkyl, Alkylsulfinylalkyl, Alkylsulfonylalkyl, für gegebenenfalls substituiertes Aralkyl oder für gegebenenfalls substituiertes Aryl steht,
n für die Zahlen 0, 1 oder 2 steht,
$R^3$ für Cyano, für einen Rest

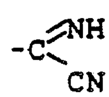

oder für einen Rest $-\underset{\underset{O}{\|}}{C}-Y-R^4$ steht und
Ar für substituiertes Phenyl oder für gegebenenfalls substituiertes Pyridyl steht, wobei
Y für Sauerstoff, Schwefel oder einen Rest $-\underset{|}{N}-R^5$ steht
$R^4$ für Wasserstoff, für Alkyl, Hydroxyalkyl, Alkoxyalkyl, Alkylthioalkyl, Halogenalkyl, Alkenyl, Halogenalkenyl, Alkinyl oder für jeweils gegebenenfalls substituiertes Cycloalkyl, Aralkyl oder Aryl steht, für einen Rest $-A-\underset{\underset{O}{\|}}{C}-Z-R^6$ steht, oder für den Fall, daß Y für Sauerstoff, Schwefel oder einen Rest $\underset{\underset{SO_2-R^7}{|}}{-N-}$ steht, auch für ein salzartig gebundenes Kation steht,
$R^5$ für Wasserstoff, für Alkyl, Hydroxyalkyl, Alkoxyalkyl, Alkylthioalkyl, Halogenalkyl, Alkenyl, Alkinyl oder Halogenalkenyl, für jeweils gegebenenfalls substituiertes Cycloalkyl, Aralkyl oder Aryl steht oder für den Rest $-SO_2-R^7$ steht,
A für einen zweifach verknüpften Alkenylrest steht,
Z für Sauerstoff, Schwefel oder einen N-Alkylrest steht,

$R^6$ für Wasserstoff, Alkyl, Alkenyl oder Alkinyl steht.
$R^7$ für jeweils gegebenenfalls substituiertes Alkyl, Aralkyl oder Aryl steht,
dadurch gekennzeichnet,

(a) daß man zum Erhalt der substituierten 1-Arylpyrazole der Formel (Ia),

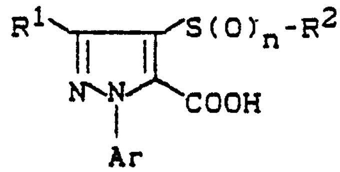

(Ia)

in welcher
$R^1$,$R^2$Ar und n die oben angegebene Bedeutung haben,
5-Halogen-1-arylpyrazole der Formel (II),

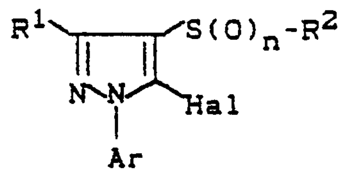

(II)

in welcher
$R^1$,$R^2$,Ar und n die oben angegebene Bedeutung haben und
Hal für Halogen steht,
mit Kohlendioxid in Gegenwart einer Lithium-organischen Verbindung und in Gegenwart eines Verdünnungsmittels umsetzt;

(b) oder daß man zum Erhalt der substituierten 1-Arylpyrazole der Formel (Ib),

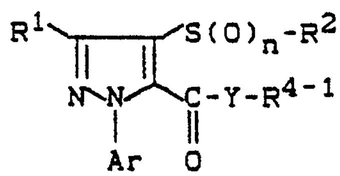

(Ib)

in welcher
$R^1$,$R^2$,Ar,Y und n die oben angegebene Bedeutungn haben und
$R^{4-1}$ für Alkyl, Hydroxyalkyl, Alkoxyalkyl, Alkylthioalkyl, Halogenalkyl, Alkenyl, Halogenalkenyl, Alkinyl, für jeweils gegebenenfalls substituiertes Cycloalkyl, Aralkyl oder Aryl steht oder für einen Rest $-A-\underset{\underset{O}{\|}}{C}-Z-R^6$ steht,

wobei
$R^6$, A und Z die oben angegebene Bedeutung haben, und außerdem für den Fall, daß Y für Schwefel oder einen Rest $-\underset{|}{N}-R^5$ steht,
auch für Wasserstoff steht;
die nach Verfahren (a) erhältlichen substituierten 1-Arylpyrazole der Formel (Ia),

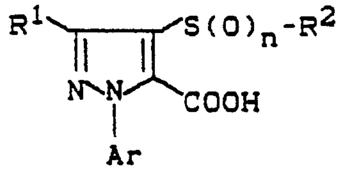

(Ia)

in welcher

$R^1$,$R^2$,Ar und n die oben angegebene Bedeutung haben,
mit Alkoholen, Aminen oder Thiolen der Formel (III),

$R^{4-1}$-Y-H (III)

in welcher
$R^{4-1}$ und Y die oben angegebene Bedeutung haben,
gegebenenfalls in Gegenwart eines Reaktionshilfsmittels und gegebenenfalls in Gegenwart eines Verdünnungsmittels sowie gegebenenfalls in Gegenwart eines Säurebindemittels umsetzt;

(c) oder daß man zum Erhalt der substituierten 1-Arylpyrazole derFormel (Ic),

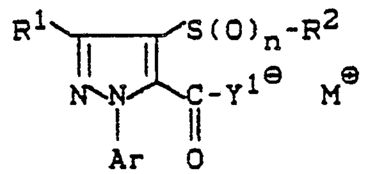

(Ic)

in welcher
$R^1$,$R^2$,Ar und n die oben angegebene Bedeutung haben,
$Y^1$ für Sauerstoff, Schwefel oder einen Rest
-N-
|
S O $_2$-$R^7$ steht und
$M^{\oplus}$ für ein anorganisches oder organisches Kation steht,
die nach Verfahren (a) oder (b)erhältlichen substituierten 1-Arylpyrazole der Formel (Ia1),

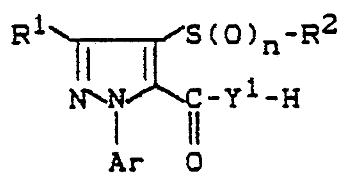

(Ia1)

in welcher
$R^1$,$R^2$,Ar,$Y^1$ und n die oben angegebene Bedeutung haben
mit anorganischen oder organischen Basen gegebenenfalls in Gegenwart eines Verdünnungsmittels umsetzt;

(d) oder daß man zum Erhalt der substituierte 1-Arylpyrazole der Formel (Id),

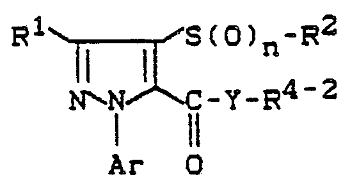

(Id)

in welcher
$R^1$,$R^2$,Ar,Y und n die oben angegebene Bedetungen haben und
$R^{4-1}$ für Alkyl, Hydroxyalkyl, Alkoxyalkyl, Alkylthioalkyl, Halogenalkyl, Alkenyl, Halogenalkenyl, Alkinyl, für jeweils gegebenenfalls substituiertes Cycloalkyl, Aralkyl oder Aryl steht oder für einen Rest -A- C(=O) -Z-$R^6$ steht, wobei $R^6$, A und Z die oben angegebene Bedeutung haben,
die nach Verfahren (c) erhältlichen substituierten 1-Arylpyrazole der Formel (Ic),

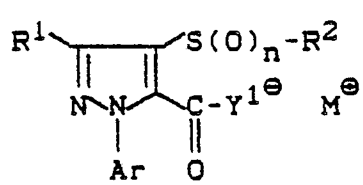

(Ic)

in welcher
$R^1$,$R^2$,Ar,$Y^1$,$M^{\oplus}$ und n die oben angegebene Bedeutung haben,
mit Alkylierungsmitteln der Formel (IV),

$R^{4-2}$-E (IV)

in welcher
$R^{4-2}$ die oben angegebene Bedeutung hat und
E für eine elektronenanziehende Abgangsgruppe steht,
gegebenenfalls in Gegenwart eines Verdünnungsmittels umsetzt;

(e) oder daß man zum Erhalt der substituierten 1-Arylpyrazole der Formel (Ie),

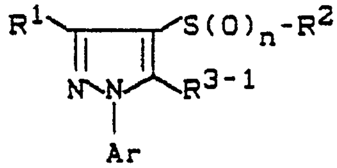 (Ie)

in welcher
$R^1$,$R^2$,Ar und n die oben angegebene Bedeutung haben und
$R^{3-1}$ für Cyano oder für einen Rest

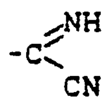

steht,
5-Halogen-1-arylpyrazole der Formel (II),

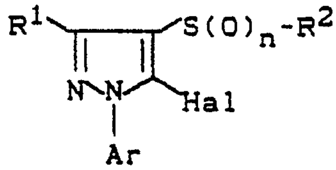 (II)

in welcher
$R^1$,$R^2$,Ar und n die oben angegebene Bedeutung haben,
mit Dicyan in Gegenwart einer Lithium-organischen Verbindung und in Gegenwart eines Verdünnungsmittels umsetzt;

(f) oder daß man zum Erhalt der substituierten 1-Arylpyrazole der Formel (If),

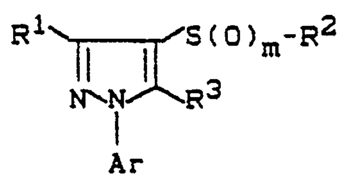 (If)

in welcher
$R^1$,$R^2$,$R^3$ und Ar die oben angegebene Bedeutung haben und
m für eine Zahl 1 oder 2 steht,
die nach Verfahren (a), (b), (c), (d) oder(e) erhältlichen substituierten 1-Arylpyrazole der Formel (Ig),

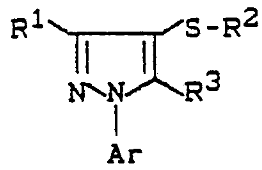

(Ig)

in welcher

$R^1$,$R^2$,$R^3$ und Ar die oben angegebene Bedeutung haben,

mit Oxidationsmitteln gegebenenfalls in Gegenwart eines Verdünnungsmittels und gegebenenfalls in Gegenwart eines Katalysators oxidiert.

7. Schädlingsbekämpfungsmittel, gekennzeichnet, durch einen Gehalt an mindestens einem substituierten 1-Arylpyrazol der Formel (I).

8. Verfahren zur Bekämpfung von Insekten, dadurch gekennzeichnet, daß man substituierte 1-Arylpyrazole der Formel (I) auf Insekten und/oder ihren Lebensraum einwirken läßt.

9. Verwendung von substituierten 1-Arylpyrazolen der Formel (I) zur Bekämpfung von Insekten.

10. Verfahren zur Herstellung von Insektiziden Mitteln, dadurch gekennzeichnet, daß man substituierte 1-Arylpyrazole der Formel (I) mit Streckmitteln und/oder oberflächenaktiven Mitteln vermischt.

Europäisches Patentamt

# EUROPÄISCHER RECHERCHENBERICHT

Nummer der Anmeldung

EP 88104950.6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A,P | DE - A1 - 3 606 476 (BAYER AG)<br>* Zusammenfassung *<br>-- | 1,6-10 | C 07 D 231/14<br>C 07 D 401/04<br>A 01 N 43/56 |
| D,A | EP - A2 - 0 201 852 (BAYER AG)<br>* Zusammenfassung *<br>---- | 1,6-10 | |

| RECHERCHIERTE SACHGEBIETE (Int. Cl.4) |
|---|
| C 07 D 231/00<br>C 07 D 401/00 |

Der vorliegende Recherchenbericht wurde für alle Patentansprüche erstellt.

| Recherchenort | Abschlußdatum der Recherche | Prüfer |
|---|---|---|
| WIEN | 30-06-1988 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument